(19) Europäisches Patentamt / European Patent Office / Office européen des brevets

(11) EP 1 381 629 B1

(12) **EUROPÄISCHE PATENTSCHRIFT**

(45) Veröffentlichungstag und Bekanntmachung des Hinweises auf die Patenterteilung:
**10.09.2008 Patentblatt 2008/37**

(21) Anmeldenummer: **01980305.5**

(22) Anmeldetag: **30.08.2001**

(51) Int Cl.:
*C07K 14/78* (2006.01) *C07K 14/705* (2006.01)
*C07K 16/28* (2006.01) *C07K 16/18* (2006.01)
*C12N 5/10* (2006.01) *C12N 15/86* (2006.01)
*G01N 33/50* (2006.01) *A61K 48/00* (2006.01)

(86) Internationale Anmeldenummer:
**PCT/EP2001/010016**

(87) Internationale Veröffentlichungsnummer:
**WO 2002/020563 (14.03.2002 Gazette 2002/11)**

(54) **REZEPTOR DER EDb-FIBRONEKTIN-DOMÄNE (II)**

RECEPTOR IN THE EDb FIBRONECTIN DOMAIN

RECEPTEUR DU DOMAINE DE LA EDb-FIBRONECTINE

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **07.09.2000 DE 10045803**
**02.05.2001 DE 10123133**

(43) Veröffentlichungstag der Anmeldung:
**21.01.2004 Patentblatt 2004/04**

(73) Patentinhaber: **Bayer Schering Pharma Aktiengesellschaft**
**13353 Berlin (DE)**

(72) Erfinder:
- **MENRAD, Andreas**
  **16515 Oranienburg (DE)**
- **REDLITZ, Alexander**
  **10961 Berlin (DE)**
- **KOPPITZ, Marcus**
  **10115 Berlin (DE)**
- **EGNER, Ursula**
  **10777 Berlin (DE)**
- **BAHR, Inke**
  **10999 Berlin (DE)**

(56) Entgegenhaltungen:
**WO-A-99/58570**

- **ARGRAVES W S ET AL: "AMINO ACID SEQUENCE OF THE HUMAN FIBRONECTIN RECEPTOR" JOURNAL OF CELL BIOLOGY, ROCKEFELLER UNIVERSITY PRESS, NEW YORK, US, US, Bd. 105, 1987, Seiten 1183-1190, XP002912334 ISSN: 0021-9525 -& DATABASE SWALL [Online] 1. November 1988 (1988-11-01) "Integrin beta-1 precursor" Database accession no. P05556 XP002208041**
- **TAKADA Y ET AL: "THE PRIMARY STRUCTURE OF THE VLA-2/COLLAGEN RECEPTOR ALPHA2 SUBUNIT (PLATELET GPIA): HOMOLOGY TO OTHER INTEGRINS AND THEPRESENCE OF A POSSIBLE COLLAGEN-BINDING DOMAIN" JOURNAL OF CELL BIOLOGY, ROCKEFELLER UNIVERSITY PRESS, NEW YORK, US, US, Bd. 109, Nr. 1, 1. Juli 1989 (1989-07-01), Seiten 397-407, XP000611310 ISSN: 0021-9525**
- **GARCIA-VELASCO JUAN A ET AL: "Regulation of monocyte chemotactic protein-1 expression in human endometrial stromal cells by integrin-dependent cell adhesion." BIOLOGY OF REPRODUCTION, Bd. 61, Nr. 2, August 1999 (1999-08), Seiten 548-552, XP001098157 ISSN: 0006-3363**
- **WAGNER CHRISTOF ET AL: "Differentiation of polymorphonuclear neutrophils in patients with systemic infections and chronic inflammatory diseases: Evidence of prolonged life span and de novo synthesis of fibronectin." JOURNAL OF MOLECULAR MEDICINE (BERLIN), Bd. 78, Nr. 6, 2000, Seiten 337-345, XP002214835 ISSN: 0946-2716**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 381 629 B1

- CASTELLANI P ET AL: "THE FRIBRONECTIN ISOFORM CONTAINING THE ED-B ONCOFETAL DOMAIN: A MARKER OF ANGIOGENESIS" INTERNATIONAL JOURNAL OF CANCER, NEW YORK, NY, US, Bd. 59, Nr. 5, 1. Dezember 1994 (1994-12-01), Seiten 612-618, XP000960445 ISSN: 0020-7136
- HASHIMOTO-UOSHIMA MARIKO ET AL: "The alternatively spliced domains EIIIB and EIIIA of human fibronectin affect cell adhesion and spreading." JOURNAL OF CELL SCIENCE, Bd. 110, Nr. 18, 1997, Seiten 2271-2280, XP002214836 ISSN: 0021-9533 in der Anmeldung erwähnt

## Beschreibung

Die Erfindung beschreibt ein Protein, das spezifisch an die $ED_b$-Fibronektin-Domäne bindet.

Fibronektine sind eine wichtige Klasse von Matrix-Glykoproteinen. Ihre Hauptrolle besteht darin, das Anhaften von Zellen an eine Vielzahl verschiedener extrazellulärer Matrizes zu ermöglichen. Das Vorhandensein von Fibronektinen auf der Oberfläche von nicht-transformierten Zellen in Kultur sowie deren Abwesenheit bei transformierten Zellen führte zur Identifizierung von Fibronektinen als wichtigen Adhäsionsproteinen. Sie wechselwirken mit zahlreichen verschiedenen anderen Molekülen, z. B. Collagen, Heparansulfat-Proteoglykanen und Fibrin, und regulieren damit die Zellform und den Aufbau des Zytoskeletts. Weiterhin sind sie verantwortlich für Zellmigration und Zelldifferenzierung während der Embryogenese. Sie sind außerdem wichtig für die Wundheilung, bei der sie die Wanderung von Makrophagen und anderen Immunzellen in das betroffene Gebiet ermöglichen, und bei der Bildung von Blutgerinnseln, indem sie das Anheften von Blutplättchen an beschädigte Regionen der Blutgefäße ermöglichen.

Fibronektine sind Dimere zweier ähnlicher Peptide, wobei jede Kette ungefähr 60 - 70 nm lang ist. Wenigstens 20 verschiedene Fibronektin-Ketten sind identifiziert worden, von denen alle durch alternatives Spleißen des RNA-Transkripts eines einzelnen Fibronektin-Gens erzeugt werden. Eine Analyse von proteolytischen Verdauen von Fibronektin zeigt, daß die Polypeptide aus sechs stark gefalteten Domänen bestehen, von denen jede Domäne wiederum sog. Wiederholungssequenzen ("repeats") enthält, deren Ähnlichkeiten hinsichtlich ihrer Aminosäuresequenz eine Klassifikation in drei Typen erlauben (Typ I, II, III). Die zentrale Region beider Ketten aus dem Dimer besteht aus einem Abschnitt von sog. Typ-III-Wiederholungen, die durchschnittlich 90 Aminosäuren lang sind (Komblihtt AR, Vibe-Pedersen K und Baralle FE, 1983. Isolation and characterization of cDNA clones for human and bovine fibronectins. Proc Natl Acad Sci USA, 80, 3218-22). Strukturelle Studien haben ergeben, daß jede Typ-III-Wiederholung aus sieben beta-Strängen besteht, die in zwei antiparallele Faltblätter gefaltet sind, wobei kurze Loop-Regionen als potentielle Protein-Protein-Wechselwirkungsstellen exponiert sind (Leahy DJ, Hendrickson WA, Aukhil I und Erickson HP. 1992. Structure of fibronectin type III domain from tenascin phased by MAD analysis of the selenomethionyl protein. Science, 258, 987-91). Diese Wiederholungen vom Typ III ermöglichen es, daß Fibronektine als Adhäsionsmoleküle fungieren, die mit Zelloberflächenmolekülen, den so."Integrinen" interagieren. Der Begriff des Integrins wurde 1987 erstmals in einem Übersichtsartikel (Hynes R.O., 1987, Cell 48, 549-550) verwendet, um eine verwandte Gruppe von heterodimeren Zelloberflächemolekülen zu beschreiben, die als Vermittler zwischen der extrazellulären Matrix und dem intrazellulären Cytoskelett fungieren und so die Zelladhäsion und Migration induzieren. Diese heterodimeren Rezeptoren "integrieren" oder vermitteln also Signale vom extrazellulären Milieu mit spezifischen zellulären Funktionen. Bis heute sind 17 beta-Untereinheiten bekannt, die mit mehr als 20 alpha-Untereinheiten spezifisch und nicht-kovalent interagieren können, um so mehr als 20 verschiedene Familien zu bilden (Plow E. F. et al. 2000, J Biol Chem, 275, 21785-21788). Insbesondere vermittelt die Sequenz RGDS, die sich in der zehnten Wiederholung vom Typ III des Fibronektins befindet (III-10), die Wechselwirkung von Fibronektin mit wenigstens 8 verschiedenen Integrinen. Darüber hinaus wurde gezeigt, daß mindestens vier Integrine in einer RGDS-unabhängigen Weise spezifisch mit Fibronektin interagieren können (Plow E. F. et al. 2000, J Biol Chem, 275, 21785-21788). Die Gruppe der Wiederholungssequenzen vom Typ III umfaßt neben den III7-, III8-, III9- und III10-Sequenzen auch die Repeats EIIIB und EIIIA ($ED_b$ und $ED_a$). Die Funktionen dieser beiden Wiederholungssequenzen sind bislang nicht oder nur zu einem geringen Maße verstanden. Eine Studie von Jamagin W. et al. (Jarnagin W. Rockey D. Koteliansky V. Wang S. und Bissell D. 1994, Expression of variant fibronectins in wound healing: cellular source and biological activitiy of the EIIIA segment in rat hepatic fibrogenesis. J Cell Biol, 127, 2037-4.8) legt nahe, daß die $ED_a$-Domäne bei einer frühen Antwort der Leber auf eine Verletzung beteiligt ist, und weiterhin scheint die $ED_a$-Domäne beim Vermitteln von Zelladhäsionsvorgängen beteiligt zu sein. Eine Fibronektin-Isoform, die die $ED_b$-Sequenz ($ED_b$-FN oder ED-B oder EDB) enthält, ist in normalem Erwachsenengewebe nicht nachweisbar, zeigt jedoch eine starke Expression in fötalem Gewebe sowie Tumorgewebe, ebenso wie auch während der Wundheilung.

Während der Entwicklung eines Tumors wird die extrazelluläre Matrix des Gewebes, in dem der Tumor wächst, durch proteolytischen Abbau bereits existierender Matrix-Bestandteile umgebaut. Hierbei entsteht eine tumorinduzierte extrazelluläre Matrix, die sich von der von normalen Geweben unterscheidet, eine geeignetere Umgebung für das Tumorwachstum bietet und die Angiogenese fördert. Angiogenese ist einer der wichtigsten Vorgänge beim Tumorwachstum und bezeichnet den Prozeß, bei dem neue Gefäße aus existierenden Endothel-beschichteten Gefäßen hervorgehen. Angiogenese ist ein invasiver Prozeß, der eine Proteolyse der extrazellulären Matrix, Proliferation, gerichtete Wanderung und Differenzierung von Endothelzellen in neue Kapillaren erfordert, die das Wachstum eines Tumors über eine bestimmte Größe hinaus unterstützen.

$ED_b$-Fibronektin ist mit dem Tumorwachstum in Verbindung gebracht worden. Weiterhin ist $ED_b$-FN um neue Blutgefäße während angiogener Vorgänge angereichert und stellt somit einen Marker für die Angiogenese bereit (Castellani P, Viale G, Dorcaratto A, Nicolo G, Kaczmarek J, Querze G, Zardi L (1994) Int. J. Cancer 59:612-618).

Die $ED_b$-Domäne ist eine Wiederholungssequenz vom Typ III, umfassend 91 Aminosäuren, und weist eine extrem hohe Sequenzhomologie zwischen dem Ratten- und Hühner-Fibronektin auf, die zwischen 96 % und 100 % liegt. Innerhalb der Domäne kommen keine RGDS- oder andere Aminosäuresequenzen vor, von denen bekannt ist, daß sie eine Interaktion mit Integrinen vermitteln. Die genaue Funktion der ED-B -Domäne ist bis dato unbekannt. Drei Studien sind veröffentlicht worden, die Spekulationen über eine allgemeine fördernde Funktion hinsichtlich Adhäsion/ Zellausbreitung für verschiedene Zellen anstellen.

Chen und Culp (1996), Exp. Cells Res. 223, 9-19, haben gezeigt, daß zelluläre Fibronektine die $ED_b$-Domäne und benachbarte Wiederholungssequenzen vom Typ III als u. U. adhäsionsfördemde Sequenzen enthalten, die von den Zellen durch alternatives Spleißen des primären Transkripts von Fibronektin reguliert werden können.

In einer späteren Studie (Chen und Culp, 1998, Clin. Exp. Metast., 16, 1, 30-42) konnte gezeigt werden, daß $ED_b$ Zell-Signal-Ereignisse induziert, die zu einer Tyrosin-Phosphorylierung von fokalen Adhäsionsproteinen führt, und zwar mit einem Mechanismus, der sich von demjenigen unterscheidet, der durch die Wiederholungssequenzen III8-9-10 vermittelt wird, welche Integrine erkennen. Es wird zunehmend anerkannt, daß die Zelladhäsion an extrazelluläre Matrizes bzw. an andere Zellen eine wichtige Quelle für Zell-Signale ist, die für die Regulation vieler Phänomene verantwortlich ist, wie z. B. Zellwachstum, -differenzierung und - transformation. Ein Adhäsions-induziertes Signalgeben beinhaltet die Aktivierung von Protein-Tyrosin-Kinasen und eine Kaskade der Tyrosin-Phosphorylierung verschiedener Signal-Moleküle. Die Autoren der eben genannten Studie weisen darauf hin, daß für diesen Signalprozeß die 125 kDa fokale Adhäsionskinase (FAK) von zentraler Bedeutung ist, die die Zell-Wechselwirkung mit Matrix-Proteinen an die Aktivierung von intrazellulären Signalmolekülen verknüpft, wie etwa Src (Xing Z, Chen HC, Nowlen JK, Taylor SJ, Shalloway D und Guan JL, 1994, Direct interaction of v-Src with the focal adhesion kinase mediated by the Src SH2 domain. Mol Biol Cell. 5, 413-21), Grb2 (Schlaepfer DD, Hanks SK, Hunter T und van der Geer P, 1994, Integrin-mediated signal transduction linked to Ras pathway by GRB2 binding to focal adhesion kinase. Nature, 372, 786-91) und PI-3-Kinase (Chen HC und Guan JL, 1994, Association of focal adhesion kinase with its potential substrate phosphatidylinositol 3-kinase. Proc Natl Acad Sei USA, 91, 10148-52). Von einem anderen fokalen Adhäsionsprotein p130cas wird ebenso angenommen, daß es bei Adhäsions-vermittelten Signalereignissen und bei spezifischen Onkogen-Aktivitäten beteiligt ist, obwohl seine genaue Funktion bisher nicht aufgeklärt ist (Sakai R. Iwamatsu A. Hirano N, et al. 1994, A novel signaling molecule, p130, forms stable complexes in vivo with v-Crk and c-Src in a tyrosine phosphorylation-dependent manner. EMBO J. 13, 3748-56; Petch LA, Bockholt SM, Bouton A, Parsons JT und Burridge K, 1995, Adhesion-induced tyrosine phosphorylation of the p130 SRC substrate. J Cell Sci, 108, 1371-9; Polte TR und Hanks SK, 1995, Interaction between focal adhesion kinase and Crk-associated tyrosine kinase substrate p130Cas. Proc Natl Acad Sci USA, 92, 10678-82).

Die Studie von Chen und Culp (1998, aaO) zeigt, daß das Mono-Wiederholungs-Protein $ED_b$ stärker fördernd war für die Ausbreitung von Balb/c 3T3-Zellen sowie für das Induzieren von FAK-Tyrosin-Phosphorylierung als die benachbarten Repeats III8 etc.. Es wird die Vermutung angestellt, daß bei physiologischen Konzentrationen der zellulären Fibronektine die Bindung des Tetrapeptids RGDS aus III10 an die Integrine möglicherweise ein nicht hinreichend starkes Signal für die Zelladhäsion erzeugt, so daß es bei dem durch die Wechselwirkung zwischen III10 und Integrin-vermittelten Mechanismen zu keiner Tyrosin-Phosphorylierungs-Antwort kommt. Es wird weiter vermutet, daß der Unterschied hinsichtlich der Antwort auf die unterschiedlichen vermittelten Zelladhäsionen durch eine unterschiedliche Aktivierung verschiedener kleiner GTP-bindender Proteine verursacht wird. Drei dieser Proteine, cdc42, rac und rho, die alle Mitglieder der ras-Superfamilie sind, spielen wichtige Rollen bei zell-morphologischen Veränderungen. cdc42 agiert sequenziell stromaufwärts von rac und induziert direkt das Erscheinen von Filopodien (Nobes CD und Hall A, 1995, Rho, rac, und cd42 GTPases regulate the assembly of multimolecular focal complexes associated with actin stress fibers, lamellipodia, and filopodia, Cell. 81, 53-62). Die Aktivierung von rac ist dann verantwortlich für die Bildung von Lamellipodien und das Netzwerk von Aktin-Filamenten zwischen den Filopodien. Weiter stromabwärts rho kann durch rac aktiviert werden und induziert fokale Adhäsionen und Aktin-Zugfasem. Alle diese Ereignisse sind abhängig von der Aktivierung von Tyrosin-Kinase, und von FAK wird angenommen, daß sie bei diesen Vorgängen beteiligt ist. Chen und Culp stellen die Vermutung an, daß die morphologischen Unterschiede zwischen Zellen, die über 7-$ED_b$-8 adhärent sind, sowie Zellen, die über 8-9-10 adhärent sind, auf der unterschiedlichen Aktivierung der kleinen GTP-bindenden Proteine beruht. Daraus wird gefolgert, daß eine Adhäsion über 8-9-10 über den Integrin-vermittelten Signalweg schließlich zu einer Aktivierung von rho führt, um fokale Adhäsionen und Aktin-Zugfasem zu erzeugen, während die Adhäsion von Balb/c-3T3-Zellen über 7-$ED_b$-8 nur zu einer Aktivierung von cdc42- und rac-Proteinen führt, nicht jedoch rho aktiviert. Für die eben genannten Mutmaßungen werden jedoch in keiner der beiden Studien Daten präsentiert.

Eine andere Studie (Hashimoto-Uoshima et al., 1997, J. Cell Sci. 110, 2271-2280) zeigt, daß die Zell-Adhäsion von kultivierten Fibroblasten durch die Anwesenheit von Fibronektin-Fragmenten, die die $ED_b$-Fibronektin-Domäne einschließen, verstärkt wird. Daraus wird gefolgert, daß die gespleißte $ED_b$-Domäne eine wichtige biologische Funktion hinsichtlich der Verstärkung der Zell-Adhäsion und - ausbreitung haben kann. Demgegenüber verhindert der Einschluß von $ED_a$ in Fragmenten in Abwesenheit von $ED_b$ die Ausbildung von guten fokalen Adhäsionen in Zellen. Darauf basierend, spekulieren die Autoren dieser Studie, daß der Einschluß beider Domänen im Fibronektin-Molekül einen Mechanismus darstellen kann, mit dem eine Zell-Adhäsion in dem Ausmaße erreicht wird, daß Fortbewegungsvorgänge erleichtert werden, bei denen sowohl Adhäsion als auch Verluste der Adhäsion für das Fortbewegen von Zellen benötigt wird.

Untersuchungen an Hühnerembryonen und erwachsenen Mäusen zeigten, daß $ED_b$-vermittelte Angiogenese durch Inhibition des Endothelzell-Integrins α3ß1 blockiert werden kann (Renato et al., AACR 2001, LB-60).

Keine der genannten Studien und Untersuchungen ergibt jedoch eine klare Antwort hinsichtlich der Funktion der $ED_b$-Domäne, noch werden Aussagen getroffen über die Identität eines möglichen (möglicher) Rezeptors (Rezeptoren) für die $ED_b$-Domäne.

Es ist daher eine Aufgabe der vorliegenden Erfindung, die Funktion der $ED_b$-Domäne weiter aufzuklären. Es ist eine weitere Aufgabe der vorliegenden Erfindung, einen möglichen spezifischen Rezeptor für die $ED_b$-Domäne zu identifizieren. Es ist eine weitere Aufgabe der vorliegenden Erfindung, den $ED_b$-spezffischen Adhäsions-Mechanismus und die Wechselwirkung mit Rezeptormolekülen aufzuklären, die beim Prozeß der Angiogenese beteiligt sein könnten. Weiterhin ist es eine Aufgabe der vorliegenden Erfindung, die für die spezifische Bindung verantwortliche $ED_b$-Region zu identifizieren.

Beschrieben wird
ein Protein,

a) das die Fähigkeit aufweist, spezifisch an die $ED_b$-Fibronektin-Domäne zu binden;

b) das spezifisch in Endothelzellen exprimiert oder aktiviert ist;

c) das spezifisch in stromalen Zellen eines Tumors exprimiert oder aktiviert ist;

d) das spezifisch in Tumorzellen exprimiert oder aktiviert ist;

e) dessen Bindung an die $ED_b$-Fibronektin-Domäne durch ein Polypeptid inhibiert wird; und

f) das ein apparentes Molekulargewicht von 120 -130 kDa für die leichte Kette und 150 -160 kDa für die schwere Kette, ermittelt durch SDS-Polyacrylamid-Gelelektrophorese, aufweist.

Insbesondere bevorzugt ist ein Protein,

a) das die Fähigkeit aufweist, spezifisch an die $ED_b$-Fibronektin-Domäne zu binden, wobei die Bindungsregion durch mindestens eine Sequenz charakterisiert ist, die ausgewählt ist aus der Gruppe, die SEQ ID NO: 1 - 3 umfaßt;

b) das spezifisch in Endothelzellen exprimiert oder aktiviert ist;

c) das spezifisch in stromalen Zellen eines Tumors exprimiert oder aktiviert ist;

d) das spezifisch in Tumorzellen exprimiert oder aktiviert ist;

e) dessen Bindung an die $ED_b$-Fibronektin-Domäne durch ein Polypeptid inhibiert wird, das eine Sequenz umfaßt, die ausgewählt ist aus der Gruppe, die SEQ ID NO:1 - 3 umfaßt; und

f) das ein apparentes Molekulargewicht von 120 -130 kDa für die leichte Kette und 150 -160 kDa für die schwere Kette, ermittelt durch SDS-Polyacrylamid-Gelelektrophorese, aufweist.

Ganz besonders bevorzugt ist ein Protein,

a) das die Fähigkeit aufweist, spezifisch an die $ED_b$-Fibronektin-Domäne zu binden, und das die α2ß1-Kette des Integrins umfaßt;

b) das spezifisch in Endothelzellen exprimiert oder aktiviert ist;

c) das spezifisch in stromalen Zellen eines Tumors exprimiert oder aktiviert ist;

d) das spezifisch in Tumorzellen exprimiert oder aktiviert ist;

e) dessen Bindung an die $ED_b$-Fibronektin-Domäne durch ein Polypeptid inhibiert wird, und das die α-Kette des Integrins umfaßt; und

f) das ein apparentes Molekulargewicht von 120 -130 kDa für die leichte Kette und 150 - 160 kDa für die schwere Kette, ermittelt durch SDS-Polyacrylamid-Gelelektrophorese, aufweist.

In einer bevorzugten Ausführungsform sind die Endothelzellen proliferierende Endothelzellen.

In einer bevorzugten Ausführungsform sind die stromalen Zellen Tumor-Stroma-Zellen.

Beschrieben wird außerdem ein Protein, dessen spezifische Bindung an die $ED_b$-Fibronektin-Domäne die Adhäsion von Endothelzellen, Tumor-Stroma-Zellen und Tumorzellen vermittelt. Die Bindungsregion kann hier durch mindestens eine Sequenz charakterisiert sein, die ausgewählt ist aus der Gruppe, die SEQ ID NO: 1 - 3 umfaßt und insbesondere die $\alpha 2\beta 1$-Kette des Integrins umfaßt.

Beschrieben wird auch ein Protein, dessen spezifische Bindung an die $ED_b$-Fibronektin-Domäne die Proliferation, von Endothelzellen induziert. Die Bindungsregion kann hier durch mindestens eine Sequenz charakterisiert sein, die ausgewählt ist aus der Gruppe, die SEQ ID NO: 1 - 3 umfaßt und insbesondere die $\alpha 2\beta 1$-Kette des Integrins umfaßt.

Weiterhin wird ein Protein beschrieben, dessen spezifische Bindung an die $ED_b$-Fibronektin-Domäne die Proliferation, Migration und Differenzierung von Endothelzellen in eine Collagenmatrix induziert, wobei die Bindungsregion durch mindestens eine Sequenz charakterisiert ist. Die Bindungsregion kann hier durch mindestens eine Sequenz charakterisiert sein, die ausgewählt ist aus der Gruppe, die SEQ ID NO: 1 - 3 umfaßt und insbesondere die $\alpha$2ß1-Kette des Integrins umfaßt.

Beschrieben wird weiterhin ein Protein, das an die $ED_b$-Fibronektin-Domäne bindet und spezifische Signaltransduktionswege induziert, wobei mindestens ein Gen induziert wird, das für ein Protein codiert, das ausgewählt ist aus der Gruppe, die
Fokale Adhäsionskinase,
CD6-Ligand (ALCAM),
die $\alpha$-Kette des Vitronektin-Rezeptors,
die integrierte alpha 8 Untereinheit und
einen/den Vorläufer für Follistatin-verwandtes Protein
umfaßt.

Die Bindungsregion kann hier durch mindestens eine Sequenz charakterisiert sein, die ausgewählt ist aus der Gruppe, die SEQ ID NO: 1 - 3 umfaßt und insbesondere die $\alpha 2\beta 1$-Kette des Integrins umfaßt.

Es wird bevorzugt, daß bei der Induktion spezifischer Signaltransduktionswege mindestens eines der genannten Gene mindestens einfach induziert wird. Bevorzugter wird dabei mindestens eines der genannten Gene zweifach induziert.

Beschrieben wird auch ein Antikörper, der in der Lage ist, an ein Protein gemäß der vorliegenden Erfindung zu binden.

Weiterhin wird ein Antikörper beschrieben, der in der Lage ist, an ein Protein zu binden, das eine Aminosäuresequenz umfaßt, die aus der Gruppe ausgewählt ist, die SEQ ID NO:1, SEQ ID NO:2, SEQ ID NO:3 und SEQ ID NO: 4 umfaßt.

In einer bevorzugten Ausführungsform ist der Antikörper in der Lage, Effekte zu inhibieren, die für die $ED_b$-Domäne spezifisch sind.

Es wird bevorzugt, daß die Bindung und Inhibierung in vitro und/oder in vivo erfolgt.

In einer bevorzugten Ausführungsform ist der Antikörper monoklonal oder rekombinant.

In einer bevorzugten Ausführungsform ist der Antikörper ein scFv-Fragment.

Beschrieben wird auch eine Zelle, die ein Protein gemäß der vorliegenden Erfindung exprimiert.

Weiterhin wird eine Zelle beschrieben, die einen Antikörper gemäß der vorliegenden Erfindung exprimiert.

Außerdem wird ein Phage beschrieben, der einen Antikörper gemäß der vorliegenden Erfindung exprimiert.

Biechrieben wird auch ein Verfahren zum Screenen auf Verbindungen, die an einen Rezeptor der $ED_b$-Fibronektin-Domäne binden, wobei das Verfahren umfaßt:

Vergleich einer Antwort von Zellen in Anwesenheit von einer oder mehrerer dieser Verbindungen mit der Kontrollantwort besagter Zellen in Abwesenheit dieser Verbindungen, wobei die Zellen
ein Protein gemäß der vorliegenden Erfindung exprimieren oder
eine Nukleinsäure, die für dieses Protein codiert, umfassen, und

wobei die Antwort bzw. die Kontrollantwort durch einen Rezeptor der $ED_b$-Fibronektin-Domäne vermittelt wird.

In einer bevorzugten Ausführungsform umfaßt die Antwort bzw. die Kontrollantwort das Anhaften der Zellen an Oberflächen, die mit der $ED_b$-Fibronektin-Domäne oder Teilen davon beschichtet sind.

In einer bevorzugten Ausführungsform des Verfahrens umfaßt eine Bindungsregion der $ED_b$-Fibronektin-Domäne die Sequenzen SEQ ID NO:1 - 4 oder Teile davon.

Es wird bevorzugt, daß die Antwort bzw. die Kontrollantwort die Proliferation der Zellen an Oberflächen umfaßt, die mit der $ED_b$-Fibronektin-Domäne oder Teilen davon beschichtet sind.

In einer bevorzugten Ausführungsform umfaßt die Antwort bzw. die Kontrollantwort die Proliferation, Migration und Differenzierung von Endothelzellen in einer Collagenmatrix, die mit der $ED_b$-Fibronektin-Domäne oder Teilen davon versetzt wird.

Es ist bevorzugt, daß die Verbindungen ausgewählt sind aus der Gruppe, die Antikörper, Antikörperfragmente, artifizielle Antikörper, Peptide, niedermolekulare Verbindungen, Aptamere und Spiegelmere umfaßt.

In einer bevorzugten Ausführungsform sind die Antikörper rekombinante Antikörper.

Es wird bevorzugt, daß die Antikörper ausgewählt sind aus der Gruppe, die scFv und Fragmente davon umfaßt.

Beschrieben wird ebenso ein Verfahren zum Screenen auf Verbindungen, die an die $ED_b$-Fibronektin-Domäne binden, wobei das Verfahren umfaßt:

a) In-Kontakt-Bringen von Zellen mit einer festgelegten Konzentration eines Proteins, das die $ED_b$-Fibronektin-Domäne oder ein Protein mit einer der in SEQ ID NO:1 - 4 dargestellten Sequenzen umfaßt, in der Anwesenheit verschiedener Konzentrationen von einer oder mehrerer der Verbindungen; und

b) Nachweisen von Unterschieden in der Antwort der Zellen auf das Protein, das die $ED_b$-Fibronektin-Domäne oder ein Protein mit einer der in SEQ ID NO:1 -4 dargestellten Sequenzen umfaßt, in der Anwesenheit der Verbindungen im Vergleich mit der Kontrollantwort der Zellen auf das Protein, das die $ED_b$-Fibronektin-Domäne oder ein Protein mit einer der in SEQ ID NO:1 — 4 dargestellten Sequenzen umfaßt, in der Abwesenheit dieser Verbindungen, wobei die Zellen ein Protein gemäß der vorliegenden Erfindung exprimieren oder
eine Nukleinsäure, die für dieses Protein codiert, umfassen, und

wobei die Antwort bzw. die Kontrollantwort durch einen Rezeptor der $ED_b$-Fibronektin-Domäne vermittelt wird.

Dabei ist bevorzugt, daß die Antwort bzw. die Kontrollantwort das Anhaften der Zellen an Oberflächen umfaßt, die mit der $ED_b$-Fibronektin-Domäne oder Teilen davon beschichtet sind.

Monoklonale Antikörper wurden mittels Standard-Methoden der Hybridomatechnologie hergestellt und immunhistologisch auf humanem Tumor-Cryoschnitten charakterisiert (s. Fig. 13).

Exemplarisch: AK AM-EDBr-2 (murines IgG 1/kappa)

In einer bevorzugten Ausführungsform umfaßt die Antwort bzw. die Kontrollantwort die Proliferation der Zellen an Oberflächen, die mit der $ED_b$-Fibronektin-Domäne oder Teilen davon beschichtet sind.

In einer weiteren bevorzugten Ausführungsform umfaßt die Antwort bzw. die Kontrollantwort die Proliferation, Migration und Differenzierung von Endothelzellen in einer Collagenmatrix, die mit der $ED_b$-Fibronektin-Domäne oder Teilen davon versetzt wird.

Es ist bevorzugt, daß die Verbindungen ausgewählt sind aus der Gruppe, die Antikörper, artifizielle Antikörper, Antikörperfragmente, Peptide, niedermolekulare Substanzen, Aptamere und Spiegelaptamere umfaßt.

Beschrieben wird weiterhin die Verwendung einer Nukleinsäure, die für ein Protein codiert, das eine Sequenz umfaßt, die ausgewählt ist aus der Gruppe, die SEQ ID NO: 1 -4 umfaßt, zum Screenen auf Verbindungen, die an einen Rezeptor der $ED_b$-Fibronektin-Domäne oder die $ED_b$-Fibronektin-Domäne binden.

Beschrieben wird auch die Verwendung eines Proteins gemäß der vorliegenden Erfindung bzw. eines Antikörpers gemäß der vorliegenden Erfindung zum Screenen auf Verbindungen, die an einen Rezeptor der $ED_b$-Fibronektin-Domäne oder die $ED_b$-Fibronektin-Domäne binden.

Ebenso wird die Verwendung einer Zelle beschrieben , zum Screenen auf Verbindungen, die an einen Rezeptor der $ED_b$-Fibronektin-Domäne oder die $ED_b$-Fibronektin-Domäne binden.

Beschrieben wird ebenso die Verwendung einer Nukleinsäure, die für ein Protein codiert, das eine Sequenz umfaßt, die ausgewählt ist aus der Gruppe, die SEQ ID NO: 1 - 4 umfaßt, zum Entwickeln von Antikörpern oder scFv-Fusionsproteinen für diagnostische oder therapeutische Zwecke.

Beschrieben wird ebenso die Verwendung eines Proteins gemäß der vorliegenden Erfindung zum Entwickeln von Antikörpern oder scFv-Fusionsproteinen für diagnostische oder therapeutische Zwecke. Unter therapeutischem Zweck ist u. a. die anti-angiogene Behandlung mit Verbindungen, die die spezifische Interaktion zwischen $ED_b$ und dem Rezeptor inhibieren, gemeint. Die Antikörper sind hierbei sowohl gegen den Rezeptor als auch gegen $ED_b$ gerichtet, wobei die Peptide der Sequenz SEQ ID NO:1 - 3 und stabilisierte Derivate davon sowie niedermolekulare Verbindungen zur Anwendung kommen.

Beschrieben wird ebenso die Verwendung einer Zelle gemäß der vorliegenden Erfindung zum Entwickeln von Antikörpern oder scFv-Fusionsproteinen für diagnostische oder therapeutische Zwecke.

Beschrieben wird ebenso die Verwendung eines Phagen gemäß der vorliegenden Erfindung zum Entwickeln von Antikörpern oder scFv-Fusionsproteinen für diagnostische oder therapeutische Zwecke.

Beschrieben wird ebenso die Verwendung eines Proteins, das eine Sequenz umfaßt, die ausgewählt ist aus der Gruppe, die SEQ ID NO: 1- 4 umfaßt, für eine pro-angiogene Therapie.

Beschrieben wird ebenso die Verwendung eines Proteins, das eine Sequenz umfaßt, die ausgewählt ist aus der Gruppe, die SEQ ID NO: 1 - 4 umfaßt, für diagnostische Zwecke.

Beschrieben wird ebenso die Verwendung eines Proteins, das eine Sequenz umfaßt, die ausgewählt ist aus der Gruppe, die SEQ ID NO: 1- 4 umfaßt, in der Gentherapie.

Beschrieben wird ebenso die Verwendung eines Proteins, das eine Sequenz umfaßt, die ausgewählt ist aus der Gruppe, die SEQ ID NO: 1- 4 umfaßt, zum Beschichten von Oberflächen, an die Endothelzellen binden.

Dabei ist bevorzugt, daß das Beschichten in vitro oder in vivo erfolgt.

Beschrieben wird ebenso die Verwendung eines Proteins, das eine Sequenz umfaßt, die ausgewählt ist aus der Gruppe, die SEQ ID NO: 1 - 4 umfaßt, in Zellkulturen.

Beschrieben wird ebenso die Verwendung eines Proteins, das eine Sequenz umfaßt, die ausgewählt ist aus der Gruppe, die SEQ ID NO: 1 - 4 umfaßt, zusammen mit mindestens einem Transplantat.

Dabei ist bevorzugt, daß das Transplantat ausgewählt ist aus der Gruppe, die Gefäß(e), Haut, Cornea, Nieren, Leber, Knochenmark, Herz, Lungen, Knochen, Thymus, Dünndarm, Pankreas, andere innere Organe sowie Teile und Zellen davon umfaßt.

Beschrieben wird ebenso die Verwendung eines Proteins, das eine Sequenz umfaßt, die ausgewählt ist aus der Gruppe, die SEQ ID NO: 1 - 4 umfaßt, zusammen mit mindestens einem Implantat.

Dabei ist bevorzugt, daß das Implantat ausgewählt ist aus der Gruppe, die Lungenimplantate, künstliche Herzschrittmacher, künstliche Herzklappen, Gefäßimplantate, Endoprothesen, Schrauben, Schienen, Platten, Drähte, Nägel, Stäbe, künstliche Gelenke, Mammaimplantate, künstliche Schädelplatten, künstliche Zähne, Zahnfüllungen und Zahnbrücken umfaßt.

Unter "Effekten, die für die $ED_b$-Fibronektin-Domäne spezifisch sind" werden alle solche Effekte verstanden, die durch die $ED_b$-Fibronektin-Domäne, nicht aber durch EIII7, EIII8, etc. hervorgerufen werden. Ein solcher Effekt ist bspw. in Chen et al., 1998 (aaO) beschrieben, d.h. eine schnelle Tyrosin-Phosphorylierung mehrerer intrazellulärer Proteine, im Gegensatz zu der eher langsamen Phosphorylierung nach einer durch die Domänen EIII8-9-10 vermittelten Adhäsion. Unter "niedermolekularen Verbindungen" werden alle Verbindungen verstanden, deren relative Molekülmasse unter etwa 1000 - 1200 liegt. Unter "Aptameren" werden auf Nukleinsäuren aufgebaute Moleküle verstanden, die in der Lage sind, als hochspezifische Liganden für eine große Anzahl von Biomolekülen zu fungieren. Unter einer "pro-angiogenen Therapie" wird jede Therapieform verstanden, bei der die Angiogenese gefördert werden soll. Unter einer "anti-angiogenen Behandlung/Therapie" wird jede Behandlungs-/Therapieform verstanden, die auf eine Inhibierung der Angiogenese abzielt. Unter "Gentherapie" wird jede Form der Therapie verstanden, die auf die Ausschaltung einer genbedingten Fehlfunktion bzw. auf die Wiederherstellung einer normalen Genfunktion bei Erkrankungen abzielt, die durch die Eliminierung oder Bereitstellung eines Proteins zu beeinflussen sind. Sie kann das Einschleusen von Fremd-DNA in Körperzellen beinhalten, ist jedoch nicht als hierauf beschränkt anzusehen. Unter "Zellkulturen" sollen sowohl Zellkulturmedien als auch Zellkulturgefäße verstanden werden. Bevorzugt sind die Zellkulturgefäße ausgewählt aus der Gruppe, die Zellkulturflaschen, -schalen, - näpfe, -platten, Mikrotiterplatten, 96-Napf-Platten, Zellkulturkolben und Bioreaktoren umfaßt.

"Diagnostische Zwecke" sind alle Zwecke, die dem Erkennen eines Zustandes eines Organismus/Organs/einer Zelle bzw. dem Zuordnen eines aktuellen Zustandes eines Organismus/Organs/einer Zelle zu einer bestimmten Zustandskategorie (z. B. einer bestimmten Krankheit) dienen, beispielsweise kann dies der Einsatz eines Kits/chemischer Reagenzien/einer Meßeinrichtung sein, zum Bestimmen einer physikalischen Größe, wie Temperatur etc., oder einer chemischen Größe, wie Konzentration etc., soll jedoch nicht als hierauf beschränkt angesehen werden.

"Therapeutische Zwecke" sind alle Zwecke, die der Verbesserung bzw. der Heilung eines Krankheitszustandes eines Organismus/Organs/einer Zelle dienen. Unter dem Begriff "Verwendung eines Proteins zusammen mit einem Implantat" ist entweder eine zeitlich oder eine räumlich identische Verwendung gemeint. Beispielsweise können Proteinmoleküle an das Implantat bei dessen "Einbau" in den Körper befestigt sein, oder aber sie können räumlich vom Implantat getrennt, aber zur gleichen Zeit wie dem "Einbau" des Implantats verabreicht werden (Injektionen etc.).

Die Erfindung wird nunmehr detailliert anhand der folgenden Beispiele und Figuren beschrieben. Dabei zeigt:

Fig. 1 eine schematische Darstellung der in dieser Studie verwendeten Wiederholungssequenzen vom Typ III;

Fig. 2 die Resultate eines Proliferationsassay unter dem Einfluß der $ED_b$-Fibronektin-Domäne (ED-B) auf Endothelzellen bzw. menschliche Stromazellen auf verschiedenen Substraten;

Fig. 3 die Resultate eines Sprießtests (tube formation test) von Endothelzellen unter dem Einfluß von ED-B;

Fig. 4 die Resultate eines Anheftungstest, bei dem das Anheften von Endothelzellen an mit ED-B beschichteten Oberflächen getestet wurde;

Fig. 5 die Resultate eines Test, ähnlich zu dem in Fig. 4, mit der Ausnahme, daß die Zelle mit verschiedenen synthetischen Peptiden vorinkubiert wurden, deren Sequenzen Teilsequenzen der $ED_b$-Fibronektin-Domäne sind;

Fig. 6 die in Fig. 5 verwendeten Teilsequenzen der synthetischen Peptide aus der $ED_b$-Fibronektin-Domäne;

Fig. 7 die Resultate eines Anheftungstests von Endothelzellen an verschiedene synthetische ED-B-Peptide;

Fig. 8 die Lage der in Fig. 6 - 7 ermittelten synthetischen Peptide in einer Modellstruktur der Peptid-Hauptkette von ED-B;

Fig. 9 die Wirkung von der $ED_b$-Fibronektin-Domäne und eines aus Loop 5 stammenden Peptids (SEQ ID NO:2) auf die Induktion von Kapillarähnlichen Strukturen in einem Sprießtest (tube formation test);

Fig. 10 die Resultate zweier Affinitäts-Chromatographie-Läufe unter Verwendung von Fn-7-8-9 bzw. Fn-7-B-8-9 von Zell-Lysaten von Oberflächen-markierten menschlichen Hautendothelzellen;

Fig. 11 die Resultate zweier Affinitäts-Chromatographie-Läufe unter Verwendung von Fn-7-8-9 bzw. Fn-7-B-8-9 von Zell-Lysaten von Oberflächen-markierten menschlichen Haut-Stroma-Zellen.

Fig. 12 Affinitätschromatographische Reinigung des $ED_b$B-Rezeptors.

Fig. 13 Immunhistologisch charkterisierte humane Tumor-Cryoschnitte.

**Fig. 1** zeigt verschiedene, in dieser Studie verwendete rekombinante Fibronektin-Fragmente, die unterschiedliche Domänen-Struktur mit unterschiedlichen Wiederholungssequenzen vom Typ III aufweisen. Dabei umfassen Fn-7-B-8-9 die Fibronektin-Domänen 7, $ED_b$, 8 und 9, Fn-7-8-9 die Domänen 7, 8 und 9, ED-B die Domäne $ED_b$, Fn-10 die Domäne 10 und Fn-6 die Domäne 6. Diese Proteine wurden als mit einem His-Tag versehene Proteine in E. coli exprimiert und auf einer Nickel-Chelat-Sepharosesäule aufgereinigt. Die in dieser Studie verwendeten Nummembezeichnungen entsprechen den in der Literatur verwendeten. Dabei bezeichnen die Abkürzungen FN-B, ED-B, EDB und $ED_b$ alle jeweils die $ED_b$-Fibronektin-Domäne und sind als synonym anzusehen.

**Fig. 2** zeigt die Resultate eines Proliferationsassay, bei dem die Wirkung der $ED_b$-Fibronektin-Domäne (ED-B) auf die Proliferation von Endothelzellen (EC) bzw.

Stroma-Zellen (SC) untersucht wurde. 1000 Zellen pro Napf wurden in 96-Napf-Platten inkubiert. Lösliches ED-B (10 μg/l) wurde dem Medium während des Proliferationsassays zugegeben. Nach drei Tagen wurde die Zellzahl mit dem MTS-Assay bestimmt. Die Proliferation der Zellen wurde durch basischen Fibronektin-Wachstumsfaktor (bFGF) induziert. Es zeigte sich, daß ED-B keine Wirkung in Abwesenheit von bFGF hatte, und ebenso konnte keine Wirkung für die Fibronektin-Domäne 10 vom Typ III in Anwesenheit von bFGF auf die Zellen nachgewiesen werden (Daten nicht gezeigt). Eine Wirkung von ED-B auf menschliche Endothelzellen-Proliferation konnte bei Zellen festgestellt werden, die auf Gelatine ausplattiert worden waren (EC/Gelatine), ebenso bei Zellen, die auf Collagen ausplattiert worden waren (EC/Collagen), wobei jedoch letzterer Effekt nicht so deutlich war, wie beim Ausplattieren auf Gelatine. Bei menschlichen Stroma-Zellen auf Gelatine (SC/Gelatine) fand eine Proliferation bereits in Abwesenheit von bFGF statt, die deutlich über der von menschlichen Endothelzellen lag. Sie konnte durch die Zugabe von bFGF bzw. bFGF + ED-B nicht gesteigert werden. Als Maß für die Zellzahl wurde die Extinktion bei 490 nm bestimmt.

Für den Proliferationsassay wurde folgende experimentelle Methode befolgt:

Material: 96-Napf-Platte (mit flachem Boden), Nunc

Medium: MCDB 131, Pen/Strep, Amphotericin (0,25 μg/ml), Heparin (20 μg/ml), hitze-inaktiviertes FCS (5 %)

Methode:

Zellen, 500-1000 pro Napf (96-Napf-Platte) in 100 μl, werden für 3 Tage in Medium mit bFGF (1 - 3 ng/ml) oder VEGF (30 - 50 ng/ml) kultiviert. Die genaue Menge sollte für jede Charge durch Titration bestimmt werden: optimal ist die minimale Konzentration, die maximale Proliferationsstimulation erreicht. Eine Synchronisation der Zellen vor dem Experiment ist nicht notwendig, kann aber gemacht werden. Nach 3 Tagen wird die Zellzahl mit dem MTS-Kit (Promega) nach Herstellerangaben bestimmt. Es empfiehlt sich, die Absorption bei mehreren Zeitpunkten zu messen, um eine maximale Absorption im linearen Bereich zu erhalten (0,5; 1; 2; 4 Stunden).

Kontrollen:

Negative Kontrolle, kein Mitogen (keine Proliferation) (-bFGF/VEGF)
Positive Kontrolle, mit Mitogen (maximale Stimulation) (+bFGF/VEGF)

**Fig. 3** zeigt die Wirkung von ED-B auf das Sprießen von Endothelzellen aus Sphäroiden. Hierzu wurden HUVEC-Sphäroide (Human Umbilical Vein Endothelial Cells = menschliche Nabelschnur-Endothelzellen) in Collagen eingebettet und durch die Zugabe von 10 μg/ml bFGF (basic Fibroblast Growth Factor) zum Sprießen induziert in Ab- oder Anwesenheit von 6 μg/ml ED-B. Es zeigte sich, daß durch die Zugabe von bFGF alleine das Sprießen induziert und dann durch die Zugabe von ED-B weiter stimuliert werden konnte (+ bFGF + ED-B).
Für den Sprießtest (tube formation test) wurde folgende experimentelle Methode verwendet:

Material:

Methylcellulose, höchste Viskosität (Sigma)
Trypsin/EDTA für Zellkultur (Gibco)
Rundboden 96-Napf-Platten (Greiner #650185)
rekombinantes bFGF (Gibco #13256-029)
rekombinantes VEGF (R & D System)
Anti-Ratte-CD31 (RDI #RDI-CD31TLD)
Heparin (Gibco #15077-027)

Lösungen:

PBS/Antibiotika: Zellkultur-PBS, 10 x Pen/Strep, 2,5 μg/ml Amphotericin 1 % Gelatine (Difco, autoklavieren und nach Abkühlung mit Pen/Strep und Amphotericin (0,25 μg/ml) versetzen

Medium: MCDB 131, Glutamin, Pen/Strep, Amphotericin (0,25 μg/ml), Heparin (20 μg/ml), hitzeinaktiviertes FCS (10 %)

Wachstumsmedium: Medium mit 2 ng/ml bFGF und 10 ng/ml VEGF

Zellen:

HUVEC

Dermale MVEC (Passage >4)

Methode:

Endothelzellen werden mit Trypsin/EDTA abgelöst und auf 5000 Zellen/ml in Medium mit 0,24 % Methylcellulose verdünnt. Je 200 μl (1000 Zellen) werden in Näpfen einer Greiner-Platte gegeben und über Nacht inkubiert. Runde Zellhaufen (Sphäroide) werden mit einer 1 ml-Pipette mit abgeschnittener Spitze geerntet und abzentrifugiert. Sphäroide werden in 1,2 % Methylcellulose/FCS resuspendiert und mit neutralisiertem Collagengel gemischt. $ED_b$ und bFGF wurden co-polymerisiert.
Wie aus der Figur deutlich wird, erfolgt durch die Zugabe von ED-B eine deutliche Steigerung des Sprießens über das durch bFGF induzierte Maß hinaus.
**Fig. 4** zeigt die Resultate eines Adhäsionstest von Endothelzellen an Mikrotiternapf-Platten, die mit ED-B beschichtet waren. Hierzu wurden Endothelzellen aus ihrem ursprünglichen Kulturgefäß durch Trypsinisierung (Trypsin/EDTA) von ihrem Substrat gelöst und dann in Mikrotitemapf-Platten, die mit verschiedenen Konzentrationen (0, 1, 2, 3, 5, 10, 20, 40 μg/ml) ED-B beschichtet waren, inkubiert und für eine Stunde zum Anhaften belassen. Als eine Negativ-Kontrolle dienten Näpfe, die mit 1 mg/ml BSA (bovinem Serumalbumin) beschichtet waren; die Adhäsion an BSA (< 10 %) wurde substrahiert.

Das Anheften wurde durch Färbung mit Kristallviolett quantifiziert, gefolgt von einer Lyse mit SDS. Die Quantifizierung erfolgte durch Messen der Extinktion bei 595 nm. Eine in der Figur waagrecht angebrachte Linie bei $A_{595}$ nm = 1,06 zeigt die 100 %ige Adhäsion an Plasma-Fibronektin an.

Das Ergebnis dieses Versuchs zeigt an, daß die Zellen an den mit ED-B beschichteten Oberflächen anhaften, was einen Rezeptor auf der Zelloberfläche für ED-B nahelegt.

Für den Anheftungs/Adhäsionstest wurde folgende experimentelle Methode verwendet:

Lösungen:

1 % BSA (Sigma, Ethanol-präzipitiert)

2 % Serum in PBS (oder eine Trypsin-Neutralisationslösung)

Medium: MCDB 131, Pen/Strep, Amphotericin (0,25 µg/ml), Heparin (20 µg/ml), 0,1 % BSA (Sigma, Äthanol-präzipitiert)

0,1 % Kristallviolett, 2 % Glutaraldehyd in PBS, sterilfiltriert

2 % SDS

Methode:

Näpfe einer 96-Napf-Platte (Nunc) werden für eine Stunde bei 37°C mit Protein belegt. Bei kleinen Proteinen (<20 kDa) oder Peptiden empfiehlt es sich, diese auf der Platte trocknen zu lassen (über Nacht ohne Deckel unter der Sterilbank). Danach werden die Näpfe mit 1 % BSA für 1 h bei 37°C abgesättigt. Zellen werden mit 1 X Trypsin abgelöst, mit 2 % Serum zur Inaktivierung des Trypsins gewaschen, und in Medium resuspendiert. Wenn Antikörper oder Peptide getestet werden sollen, werden die Zellen in Suspension mit diesen für 30 min bei 37°C vorinkubiert. Pro Napf (96-Napf-Platte) werden $10^4$ Zellen in einem Volumen von 50 - 100 µl für 1 h bei 37°C inkubiert. Der Überstand wird vorsichtig abgegossen, die Platte zum Abtropfen auf einem Papierhandtuch für 1 min umgedreht stehen lassen und angeheftete Zellen werden mit Kristallviolett/Glutaraldehyd für 15 min gefärbt und fixiert. Die Näpfe werden 3mal mit PBS gewaschen und die Zellen anschließend durch Zugabe von 2 % SDS lysiert (15 min auf dem Schüttler). Die Absorption bei 595 nm wird gemessen. Nach dreimaligem Waschen mit Wasser können die Zellen, falls erwünscht, erneut angefärbt werden.

Kontrollen:

Negative Kontrolle: Leer-Näpfe (BSA-Kontrolle)
Positive Kontrolle: Plasma-Fibronektin (2,5 µg/ml)

$$\%\ \text{Adhäsion} = A_{595}\ (\text{Probe}) : 100 \times A_{595}\ (\text{Fibronektin})$$

**Fig. 5** zeigt die Resultate eines Test, ähnlich dem aus Fig. 4 mit der Ausnahme, daß vor dem Anheften an mit ED-B beschichteten Mikrotitemapf-Platten die Endothelzellen mit 250 µM verschiedener synthetischer Peptide vorinkubiert wurden, deren Sequenz eine Teilsequenz der $ED_b$-Fibronektin-Domäne war. Die Anheftung wurde durch die Bestimmung der Extinktion bei 595 nm ($A_{595}$) bestimmt. Die in der Figur aufgetragenen Peptidbezeichnungen werden in Fig. 6 erläutert. Dabei entspricht Peptidsequenz Nr. 043 der in SEQ ID NO:1 dargestellten Sequenz, Peptidsequenz Nr. 553 der SEQ ID NO:2, Peptidsequenz Nr. 038 der SEQ ID NO:3. Ein hoher $A_{595}$-Wert entspricht einer nicht-inhibierten Anhaftung, während ein niedriger $A_{595}$-Wert einer Inhibition der Anhaftung durch das entsprechende Peptid entspricht.

Die für Fig. 4 beschriebene Methode wurde befolgt.

**Fig. 6** zeigt die der Gesamtsequenz der $ED_b$-Fibronektin-Domäne entnommenen Teilsequenzen der synthetischen ED-B-Peptide mit den dazu gewählten Sequenzbezeichnungen. Es wird der Ein-Buchstaben-Code für Aminosäuren verwendet.

**Fig. 7** zeigt die Resultate eines Tests, ähnlich zu dem in Fig. 5, außer, daß hier die Mikrotitemapf-Platten nicht mit der $ED_b$-Fibronektin-Domäne beschichtet waren, sondern mit den sich im Test aus Fig. 5 als inhibitorisch erwiesenen Peptiden, bzw. nicht-inhibitorisch erwiesenen Peptide vor-inkubiert wurden und somit mit diesen beschichtet wurden. Dabei zeigt es sich, daß die Zellen in diesen Tests nunmehr bei einer Beschichtung mit jeweils eines der inhibitorischen Peptide Anheftung zeigen, gemessen am $A_{595}$-Wert, während ein aus Fig. 5 als nichtinhibitorisches erwiesenes Peptid zu keiner Anheftung führt.

Die für Fig. 4 beschriebene Methode wurde befolgt.

**Fig. 8** zeigt eine Modellstruktur der $ED_b$-Fibronektin-Domäne (ED-B), aus dem die Lage der inhibitorischen Peptide Nr. 1 (= SEQ ID NO:1), Nr. 2 (= SEQ ID NO:2) und Nr. 3 (= SEQ ID NO:3) hervorgeht. Es zeigt sich, daß sich diese inhibitorischen Peptide auf Loop 1 bzw. Loop 5 der ED-B-Struktur befinden und damit die Region der Domäne identifizieren, über die eine Bindung zur Zelle bzw. dem auf der Zelle befindlichen Rezeptor stattfindet. Die in Fig. 8 gezeigte Modellstruktur der ED-B-Domäne beruht auf einer bereits ermittelten Struktur der Fibronektin-Domäne 7 vom Typ III. N-T und C-T stehen für N- bzw. C-Terminus.

**Fig. 9** zeigt die Resultate eines Tests, bei dem die Wirkung der Zugabe von ED-B und dem zuvor als inhibitorisch ermittelten Peptid Nr. 2 sowie die Zugabe der Fibronektin-Domäne 6 vom Typ III auf die Induktion von Kapillar-ähnlichen Strukturen (tube formation) im Sprießtest untersucht wird. Es zeigt sich, daß über dem basalen, durch bFGF induzierten Eindringen in Collagen-Gele die größte Wirkung durch das Anheftungs-inhibitorische Peptid SEQ ID NO:2 erzeugt wird. Dieses Peptid hat also eine stimulatorische Wirkung auf das Eindringen von Endothelzellen in Collagen-Gele. Dieses Peptid entspricht daher der Bindungsregion von $ED_b$ und stimuliert, in Analogie zu $ED_b$ selbst, das Eindringen von Endothelzellen in das Collagen.

Die für Fig. 3 beschriebene Methode wurde befolgt.

**Fig. 10** zeigt die Resultate einer Affinitätschromatographie von Zell-Lysat aus Oberflächen-markierten, menschlichen Hautendothelzellen. Hierzu wurden proliferierende, an der Zelloberfläche biotinylierte Endothelzellen mit einem Detergenz lysiert und einer Affinitätschromatographie unterzogen, bei der kurze Fragmente Fibronektin mit oder ohne die eingefügte $ED_b$-Fibronektin-Domäne an Sepharose gekoppelt waren (mit der $ED_b$-Fibronektin-Domäne = Fn-7-B-8-9, ohne die $ED_b$-Fibronektin-Domäne = Fn-7-8-9). Es konnte gezeigt werden, daß ein biotinyliertes Protein mit einem apparenten Molekulargewicht von 120 - 130 kDa spezifisch an das ED-B-enthaltende Fragment bindet (siehe Pfeil). Die Elution erfolgt mittels EDTA. Es wurden mehrere, im folgenden beschriebene Fraktionen gesammelt. Die Fraktionen wurden anschließend SDS-PAGE unterzogen und mit Western-Blot mit Streptavidin-Peroxidase- und Chemolumineszenz (ECL) untersucht. Die Spuren 1 und 5 zeigen Prä-Elutions-Fraktionen, während die Spuren 2, 3, 4 bzw. 6, 7, 8 die eluierten Fraktionen 1, 2 und 3 zeigen. Spuren 1 - 4 zeigen die Chromatographie mit Fn-7-8-9, während Spuren 5 - 8 die Chromatographie mit Fn-7-B-8-9 zeigen. Das hier gezeigte Resultat ist ein starkes Indiz dafür, daß die prominente Bande mit einem Molekulargewicht zwischen 120 - 130 kDa ein Protein ist, das spezifisch an ein $ED_b$-enthattende Fibronektin-Fragment bindet und somit einen Rezeptor für die $ED_b$-Fibronektin-Domäne darstellt.

Für die Biotinylierung und Lyse der Endothelzellen wurde folgende experimentelle Methode befolgt:

Material: Biotinamidohexansäure-3-sulfo-N-Hydroxysuccinimid-Ester; Sigma PBS w/o Mg/Ca (Dulbecco)
Hepes-Puffer: 20 mM Hepes pH 7,6, 1 mM $CaCl_2$, 1 μM $MgCl_2$, 0,1 % $NaN_3$,
1 % CHAPS (VN)
und Boehringer vollständiger Mini Protease-Inhibitor, EDTA-frei, Cocktail-Tabletten

Methode: Die Zellkulturflaschen werden vor und nach dem Biotinylieren jeweils 3mal mit PBS w/Ca + Mg gewaschen. Vor dem letzten Waschvorgang wird der Biotinpuffer (1 mg/15ml PBS) angesetzt. In jede der Flaschen werden langsam 5 ml des Puffers (für 225 $cm^2$) oder 12,5 ml (500 $cm^2$ Platten) in die Mitte des Bodens pipettiert, so daß sich das Volumen über den ganzen Flaschenboden unter Schwenken verteilen kann. Dann wird die erste Kulturflasche mit der Hälfte des Lysepuffervolumens behandelt. Der Puffer wird ebenfalls in die Mitte des Flaschenbodens pipettiert und über die gesamte Oberfläche verteilt. Dann werden die Zellen mit Hilfe eines Zellschabers abgeschabt. Anschließend wird das Gesamtvolumen der 1. Kulturflasche in die 2. Flasche pipettiert, wo der Vorgang dann wiederholt wird. Nach der letzten Flasche wird das Volumen in ein 50 ml konisches Zentrifugenröhrchen überführt. Mit der anderen Hälfte des Lysepuffers wird dieser Vorgang in allen Kulturflaschen wiederholt (ohne Zellschaber) und das Endvolumen ebenfalls in Zentrifugenröhrchen gegeben. Zentrifugiert wird in 50 ml konischen Zellkulturröhrchen bei 3000 U/min, 5 min bei RT (Heraeus-Tischzentrifuge). Das Lysat wird abpipettiert und sollte idealerweise sofort für die Affinitätschromatographie eingesetzt werden (kann notfalls aber auch bei -80°C eingefroren werden).

Für die kovalente Kopplung von Proteinen an Sepharose wurde folgendes Vorgehen gewählt:

Material: aktivierte CH Sepharose 4 B Pharmacia Biotech,
Code-No. 17-0490-01

1 mmol HCl, 2,2 % $NaHCO_3$

Methode: Die HCl wird im Eisbad gekühlt, die Sepharose läßt man auf Raumtemperatur erwärmen.

Dann wird die Sepharose mit 1 mmol HCl gewaschen. Pro ml Sepharose benötigt man 10 ml HCl. Die Sepharose läßt man langsam in das vorgekühlte Röhrchen rieseln, wo sie dann für etwa 15 Minuten aufquillt. (1 g Sepharose entspricht 3 ml gequollener Sepharose). Anschließend wird das Röhrchen für 1 Min. bei 800 U zentrifugiert. Der Überstand wird abpipettiert und verworfen.

Dieser Vorgang wird 3mal wiederholt.

Nach dem 3. Waschen wird erneut HCl zugegeben, das Röhrchen wird geschwenkt und 3 - 5 Min. bei 800 U zentrifugiert. Der Überstand wird abpipettiert und das Pellet wird mit 20 ml Millipore-Wasser gelöst und in 2 neue Zentrifugenröhrchen überführt (je 1 Röhrchen für 7-EDB-8-9 Sepharose und für 7-8-9 Sepharose, d.h. Sepharose, an die ein Polypeptid mit den Repeats III7, $ED_b$, III8 und III9 bzw. III7, III8 und III9 gekoppelt ist.). Die Röhrchen werden sofort wieder abzentrifugiert, der Überstand wird abpipettiert und die 1 -5 mg Protein/ml Sepharose können gekoppelt werden.

(d. h. 2 mg Protein/ml Sepharose 7-8-9
2 mg " " 7-EDB-8-9)

Die Röhrchen werden durch Umschwenken gemischt. Dann erfolgt zügig die Zugabe von 2,2 % NaHC3. (50 µl/ml Gel). Dadurch wird die restliche HCl neutralisiert. Die Röhrchen werden umgeschwenkt und bei höchster Stufe auf einem "Wipptisch" für 1 - 5 Std. durchgemischt.

Anschließend werden die Röhrchen wieder abzentrifugiert.

Zur Bestimmung der Proteinkonzentration, die bei der kovalenten Kopplung an Sepharose eingesetzt werden soll, wurde ein Bradford-Test durchgeführt:

Material: BSA-Stammlösung 2 mg/ml
Bradford Reagenz

Methode: Die BSA-Lösung wird wie folgt auf eine Nunc-Immuno-Plate (Maxi Sorp) aufgetragen: 5µg-4µg-3µg-2µg-1 µg (80 µl Vol. + 20 µl Assay)
Vorverdünnung für BSA: 5µg/50µl = 0,1 mg/ml
Die Stammlsg. 2 mg/ml wird durch eine 1:20-Verdünnung auf eine Konzentration von 0,1 mg/ml verdünnt.

Zur Durchführung der Affinitätschromatographie bzw. zur Elution wurde folgendes Vorgehen gewählt:

a) Affinitätschromatographie

Material: aktivierte CH Sepharose 4B Pharmacia Biotech,
Code-No. 17-0490-01
Puffer A (20 mM Hepes pH 7,6, 1 mM $CaCl_2$, 1mM $MgCl_2$, 0,1 % $NaN_3$)
Puffer B (Puffer A + 150 mM NaCl + 0,1 % Chaps)
Puffer C (Puffer A + 0,1 % Chaps)
PH 4-Puffer (Millipore-Wasser + 0,1 % Eisessig + 0,1 % Chaps)
EDTA-Puffer (Puffer A + 200 mM EDTA pH 8,5 + 0,1 % Chaps)

Methode: Das Lysat wird zunächst 3 x über die Säule geschickt.

Unter der Säule befindet sich ein Röhrchen zum Auffangen der Flüssigkeit. Die ersten 2 ml des Lysats werden vorsichtig mit einer Eppendorfpipette auf das Gel gegeben. Für das weitere Lysatvolumen verwendet man eine Meßpipette. Zu beachten ist, daß die Säule gerade ist. Wird die Säule zum ersten Mal benutzt, wird vor dem eigentlichen Durchlauf ein "Trockendurchlauf" mit allen proteinfreien Puffern durchgeführt. Eine Säulenfüllung sollte maximal 5 x benutzt werden.

Wenn das Lysat eingefroren (-80°C) vorliegt, wird es zunächst im Wasserbad erwärmt und dann zentrifugiert (5 min. bei 3000 U).

Frisches Lysat ist jedoch immer dem eingefrorenen vorzuziehen.

Von dem Lysat werden 500 µl in ein Eppendorfgefäß abpipettiert.
Dies dient zur Untersuchung des Lysats vor und nach der Chromatographie..

Werden 2 Säulen verwendet (je eine für 7-8-9 Sepharose und für 7-B-8-9 Sepharose), wird jeweils die Hälfte des Lysatvolumens über jede der Säulen geschickt. Beide Säulen sollten dieselbe Flußrate haben. Ist dies nicht der Fall, wird die "langsamere" Säule entsprechend lange geschlossen. Die ideale Flußrate beträgt 0,2 - 0,5 ml/min.

Ist das Lysat 3 x durch die Säule gelaufen, wird von dem Durchlauf, nachdem er gemischt wurde, ebenfalls 500 µl in ein Eppendorfgefäß pipettiert, damit auch hier eine Untersuchung erfolgen kann.

Anschließend werden je 10 Säulenvolumina Puffer B und Puffer C über die Säule geschickt. Danach ist der Waschvorgang abgeschlossen.

b) Elution

Präelution: Puffer C wird über die Säule geschickt, damit festgestellt werden kann, ob trotz der Waschprozedur noch Proteine verblieben sind. 500 µl werden in einem Eppendorf-Gefäß aufgefangen. (Bei 2 Säulen entsprechend 2 x 500 µl).

EDTA-Elution: EDTA komplexiert die Ca- und Mg-Ionen. Dadurch werden die Endothelzell-Proteine eluiert, die Ca und Mg zur Bindung benötigen. 2 x 4 ml EDTA-Puffer werden über die Säule (bzw. über beide Säulen) geschickt und in 2 Fraktionen (E1 u. E2 / BE1 u. BE 2) in Falcon-Röhrchen aufgefangen. Dann wird der Röhrcheninhalt gemischt und 5000 µl werden in ein (bzw. 2) Eppendorfgefäß(e) abpipettiert.

pH 4-Elution: Der eigentliche pH-Wert des Puffers beträgt 3,7. Außerhalb des neutralen pH-Bereiches (pH 6 - 8) kann man die Bindung des Rezeptors an sein Protein hemmen. Auch hier werden wie bei der EDTA-Elution 2 x 4 ml PH 4-Puffer über die Säule geschickt, in 2 Fraktionen gesammelt und jeweils 500 µl abpipettiert (4,1 u. 4,1 / B 4,1 und B 4,2).

Anschließend werden 3 Säulenvolumen Puffer A über die Säule gegeben, damit die Säure herausgewaschen wird. Das letzte Säulenvolumen verbleibt in der Säule. Die Säule wird geschlossen und im Kühlschrank verwahrt.

Die 500 µl-Fraktionen in den Eppendorfgefäßen werden für mind. 15 Min. bei - 80°C eingefroren und anschließend in einem "Speed vac" gefriergetrocknet.

Die so erhaltenen Fraktionen bzw. Prä-Elutions-Fraktionen wurden mit SDS-PAGE aufgetrennt und einem Western Blot unter reduzierenden Bedingungen unterzogen.

**Fig.11** zeigt dasselbe Experiment wie in Fig. 10, mit der Ausnahme, daß hier nicht lysierte Endothelzellen, sondern lysierte Stroma-Zellen verwendet werden. In dem in **Fig. 11** gezeigten Western-Blot zeigen die Spuren 1 - 3 die Elution von einer Affinitätssäule mit Fn-7-8-9, während die Spuren 4 - 6 die Elution von einer Affinitätssäule von Fn-7-B-8-9 zeigen. Spuren 1 und 4 sind Prä-Elutionsfaktoren, während Spuren 2, 3 bzw. 5, 6 die Fraktionen 1 und 2 des jeweiligen Elutionslaufes zeigen. Eine prominente Bande mit einem apparenten Molekulargewicht von 120-130 kDa, wie sie in Fig. 10 zu sehen ist, kann bei diesem Zell-Lysat aus menschlichen Stroma-Zellen nicht festgestellt werden.

Die in der vorstehenden Beschreibung, den Ansprüchen sowie den Zeichnungen offenbarten Merkmale der Erfindung können sowohl einzeln als auch in beliebigen Kombinationen für die Verwirklichung der Erfindung in ihren verschiedenen Ausführungsformen wesentlich sein.

**Fig.12** zeigt das ED-B-Bindungsprotein, welches mittels Affinitätschromatografie, wie beschrieben, aufgereinigt und mittels SDS-Gradientengelelektrophorese (4-12%) aufgetrennt wurde. Die spezifisch angereicherten Doppel-Banden (Pfeile) wurden ausgeschnitten und mittels Massenspektroskopie analysiert.
Die Sequenzanalyse identifizierte das isolierte Protein eindeutig als das alpha2-betal-Integrin, wobei die prädominante, schwere Bande der beta1-, die leichte Bande der alpha2-Untereinheit entspricht.
Dieser Befund spricht dafür, daß die Bindung an EDB hauptsächlich durch die beta1-Untereinheit des Integrins vermittelt wird. Entsprechend des untersuchten Zelltyps können auch andere alpa-Untereinheiten (e.g. alpha2) mit beta1 kombiniert die Bindung an EDB-FN vermitteln.

**Fig. 13** zeigt immunhistologisch charakterisierte humanene Tumor-Cryoschnitten, wobei bedeuten:

A: Nierenzellkarzinom, Pfeile zeigen die spezifische Färbung mittels AK AM-EDBr-2
B: Close-up des selben Präparates
C: Hepatozelluläres Karzinom
D: Melanom (hier wurde keine spezifische Färbung gefunden)

**Analyse des EDB-Rezeptors**

Die Banden wurden aus einem 1 D-Gel ausgeschnitten, mit $NH_4HCO_3$-Lösung und Acetonitril gewaschen, getrocknet und mit Trypsinlösung zur Proteolyse der Proteine im Gel versetzt. Die aus dem Gel in die Verdaulösung eluierten Peptide wurden über $\mu C_{18}$-Säulen aufkonzentriert und entsalzt und mit MALDI-Massenspektrometrie vermessen (= Liste von Peptidmassen des verdauten Proteins).

Mit den gefundenen Peptidmassen aus jeder Gelbande wurde eine Datenbanksuche durchgeführt. Bei nicht eindeutigen Suchergebnissen wurden zusätzlich MALDI-PSD-Spektren (Fragmentspektren) eines einzelnen Peptids vermessen. Die Spektren wurden entweder direkt zur Bestätigung einer vermuteten Peptidsequenz verwendet (Interpretation des Spektrums) oder es wurde eine Datenbanksuche mit diesen Spektren durchgeführt.

Untersuchte Banden:

Bande A = Bande 1 aus Präparation 6
Bande 4 aus Präparation 5
Bande 6 aus der sauren Elution

Ergebnis: Integrin α2

- siehe Datenbanksuchergebnis Bande 4
- die Spektren aus Bande 1 und 6 zeigen die gleichen intensivsten Peptide ein PSD-Spektrum eines Peptids aus Bande 1 bestätigt eine Teilsequenz von Integrin α2

Bande B = Bande 2 aus Präparation 6
Bande 5 aus Präparation 5
Bande 7 aus der sauren Elution

Ergebnis: Integrin ß1

- siehe Datenbanksuchergebnisse Bande 5 und 7
- das Spektrum aus Bande 2 zeigt die gleichen intensivsten Peptide
- die Datenbanksuche mit einem PSD-Spektrum aus Bande 2 bestäötigte Integrin ß1

BSA

- ist in allen drei Banden enthalten
- durch die Datenbanksuche mit einem PSD-Spektrum und zahlreiche Peptidmassen bestätigt

```
function expandIt(whichE1) {whichE1.style.display = (whichE1.style.display == "none")? "":"none";}
```

**ProFound** - Search Result Summary

Version 4.10.6
© 1997-2000 ProteoMetrics

```
A:hover { COLOR: red } function toggleIt(E1) {whichIm = event.srcElement;if (E1.style.display == "none"){E1.style.display = "";whichIm.src = "/prowl/minus.gif";}else{whichIm.src = "/prowl/plus.gif";E1.style.display = "none";}} A:hover { COLOR: red }
```

**Protein Candidates** for search 20010208092948-0121-149234049162 [121056 sequences searched]

| Rank | Probability | Est'd Z | Protein Information and Sequence Analyse Tools (T) | % | pI | kDa |
|---|---|---|---|---|---|---|
| 1 | 1.0e+000 | 2.20 | gi\|4504743\|ref\|NP_002194.1\| integrin alpha 2 precursor | 19 | 5.2 | 129.28 |
| +2 | 2.3e-010 | - | gi\|628012\|pir\|\|A53933 myosin I myr 4 - rat | 15 | 9.6 | 116.17 |
| | - | - | gi\|6981242\|ref\|NP_037115.1\| unconventional myosin from rat 4 for myosin I heavy chain | 15 | 9.6 | 116.12 |
| 3 | 8.3e-011 | - | gi\|7513010\|pir\|\|T00322 hypothetical protein KIAA0542 - human | 15 | 11.5 | 117.58 |
| 4 | 1.7e-012 | - | gi\|4210973\|gb\|AAD12058.1\| (AF105016) vacuolar proton translocating ATPase 116-kDa subunit a2 isoform; V-ATPase 116-kDa isoform a2 isoform [Bos taurus] | 11 | 5.9 | 97.99 |

| | | | | | | |
|---|---|---|---|---|---|---|
| 5 | 5.4e-013 | - | gi\|543747\|sp\|P36633\|ABP_RAT AMILORIDE-SENSITIVE AMINE OXIDASE [COPPER-CONTAINING] PRECURSOR (DIAMINE OXIDASE) (DAO) (AMILORIDE-BINDING PROTEIN) (ABP) (HISTAMINASE) | 16 | 6.6 | 85.00 |
| 6 | 4.2e-013 | - | gi\|7656867\|ref\|NP_055059.1\| a disintegrin-like and metalloprotease (reprolysin type) with thrombospondin type 1 motif, 2 | 12 | 6.8 | 134.71 |
| 7 | 8.6e-014 | - | gi\|3688530\|emb\|CAA09465.1\| (AJ011035) phospholipase C beta 2 [Rattus norvegicus] | 11 | 5.8 | 134.87 |
| +8 | 6.5e-014 | - | gi\|4504085\|ref\|NP_000399.1\| glycerol-3-phosphate dehydrogenase 2 (mitochondrial) | 21 | 7.0 | 80.80 |
| - | - | | gi\|7446012\|pir\|\|G02093 glycerol-3-phosphate dehydrogenase – human | 21 | 7.3 | 80.82 |
| 9 | 5.0e-014 | - | gi\|7513725\|pir\|\|T29098 microtubule-associated protein 4, muscle-specific - mouse (fragment) | 14 | 8.1 | 114.87 |
| 10 | 4.7e-014 | - | gi\|6005970\|ref\|NP_009078.1\| zinc finger protein 175 | 22 | 9.6 | 81.59 |

NOTE:

1. To search again using unmatched masses, click the symbol ®.

2. Highly similar protein sequences were given the same rank (IE user: click "+" to expand/contract).

| Input Summary | |
|---|---|
| Date & Time | Thu Feb 08 08:29:55 2001 UTC (Search Time: 6.30 sec.) |
| Sample ID | EDB Fibronektin, Bande 4 |
| Database | NCBInr [..\databases\nr] |
| Taxonomy Category | Mammalia (mammals) |
| Protein Mass Range | 80 - 135 kDa |
| Protein pI Range | 0.0 -14.0 |
| Search for | Single protein only |
| Digest Chemistry | Trypsin |
| Max Missed Cut | 2 |
| Modifications | +C3H5ON@C(Partial); +O@M(Partial); |
| Charge State | MH+ |
| Peptide Masses (Da,Average) | |
| Tolerance(AVG) | 1.00 ppm |
| Peptide Masses (Da,Monoisotopic) | 935.536 1007.504 1179.635 1222.729 1277.731 1307.689 1473.816 1479.833 1510.835 1553.895 1567.768 1586.801 1638.888 1707.772 1819.830 1851.993 1915.959 1931.980 1947.990 1973.966 1993.998 2044.968 2051.077 2068.095 2095.065 2150.093 2224.097 2283.137 2344.115 |

2501.214 2705.123 2775.304 2872.336 2902.333 2932.502 3052.424 3280.542

**Tolerance(MON)** 50.00 ppm

**Number of Peptides** 37

ProteoMetrics' ProFound is based on ProFound at The Rockefeller University [search + transmission time: >=6.33 sec]

```
function expandIt(whichE1) {whichE1.style.display = (whichE1.style.display == "none")? "":"none";}
```

**ProFound** - Search Result Summary

Version 4.10.6

© 1997-2000 ProteoMetrics

```
A:hover { COLOR: red } function toggleIt(E1) {whichIm = event.srcElement;if (E1.style.display == "none"){E1.style.display = "";whichIm.src = "/prowl/minus.gif";}else{whichIm.src = "/prowl/plus.gif";E1.style.display = "none";}} A:hover { COLOR: red }
```

**Protein Candidates** for search 20010207110038-0035-149234049162 [121056 sequences searched]

| Rank | Probability | Est'd Z | Protein Information and Sequence Analyse Tools (T) | % | pI | kDa |
|---|---|---|---|---|---|---|
| +1 | 1.0e+000 | 1.15 | gi\|124963\|sp\|P05556\|ITB1_HUMAN FIBRONECTIN RECEPTOR BETA SUBUNIT PRECURSOR (INTEGRIN BETA-1) (CD29) (INTEGRIN VLA-4 BETA SUBUNIT) | 17 | 5.3 | 88.45 |
| | - | - | gi\|762977\|emb\|CAA33272.1\| (X15202) Fn receptor beta prechain [Mus musculus] | 11 | 5.8 | 88.18 |
| | - | - | gi\|72070\|pir\|\|IJMSFB fibronectin receptor beta chain precursor - mouse | 11 | 5.8 | 88.31 |
| | - | - | gi\|8393636\|ref\|NP_058718.1\| integrin, beta 1 | 11 | 5.8 | 88.48 |
| | - | - | gi\|124964\|sp\|P09055\|ITB1_MOUSE FIBRONECTIN RECEPTOR BETA SUBUNIT PRECURSOR (INTEGRIN BETA-1) | 11 | 5.7 | 88.21 |
| | - | - | gi\|10336839\|gb\|AAG16767.1\|AF192528_1 (AF192528) integrin beta-1 subunit [Sus scrofa] | 11 | 5.3 | 88.25 |
| | - | - | gi\|1708573\|sp\|P53712\|ITB1_BOVIN FIBRONECTIN RECEPTOR BETA SUBUNIT (INTEGRIN BETA-1) (CD29) (INTEGRIN VLA-4 BETA SUBUNIT) | 9 | 5.3 | 85.31 |
| | - | - | gi\|1708574\|sp\|P53713\|ITB1_FELCA FIBRONECTIN RECEPTOR BETA SUBUNIT PRECURSOR (INTEGRIN BETA-1) (CD29) (INTEGRIN VLA-4 BETA SUBUNIT) | 9 | 5.2 | 88.08 |
| 2 | 1.9e-004 | - | gi\|5453910\|ref\|NP_006216.1\| phospholipase C, delta 1 | 8 | 6.2 | 85.75 |
| +3 | 7.7e-005 | - | gi\|1589134\|prf\|\|2210313A phosphatidylinositol 3- | 10 | 5.9 | 83.4 |

| | | | | | | |
|---|---|---|---|---|---|---|
| | | | kinase:SUBUNIT=55kD regulatory [Rattus norvegicus] | | | 6 |
| - | | - | gi\|6981358\|ref\|NP_037137.1\| phosphoinositide 3-kinase p85 (other splicing variants: p55 and p50) | 8 | 5.9 | 83.51 |
| 4 | 1.8e-005 | - | gi\|1163174\|gb\|AAA85505.1\| (U32575) similar to yeast Sec6p, Swiss-Prot Accession Number P32844; similar to mammalian B94, Swiss-Prot Accession Number Q03169; Method: conceptual translation supplied by author [Rattus norvegicus] | 8 | 5.8 | 86.48 |
| 5 | 1.1e-005 | - | gi\|2137061\|pir\|\|PC4183 1-phosphatidylinositol phosphodiesterase (EC 3.1.4.10) delta 1 - Chinese hamster (fragment) | 10 | 5.9 | 84.62 |
| 6 | 6.1e-006 | - | gi\|9910238\|ref\|NP_064388.1\| general control of amino acid synthesis, yeast homolog-like 2 | 10 | 9.6 | 93.37 |
| 7 | 2.4e-006 | - | gi\|10047327\|dbj\|BAB13451.1\| (AB046845) KIAA1625 protein [Homo sapiens] | 6 | 9.0 | 97.20 |
| 8 | 1.1e-006 | - | gi\|5032191\|ref\|NP_005793.1\| tumor protein p53-binding protein | 10 | 9.7 | 93.48 |
| +9 | 9.9e-007 | - | gi\|9910260\|ref\|NP_064581.1\| HCNP protein | 9 | 8.7 | 98.86 |
| - | | - | gi\|6330235\|dbj\|BAA86491.1\| (AB033003) KIAA1177 protein [Homo sapiens] | 6 | 5.6 | 87.80 |
| +10 | 9.6e-007 | - | gi\|9453796\|emb\|CAB99365.1\| (AL117378) dJ131F15.2 (phospho-diesterase I/nucleotide pyrophosphatase 1 (homologous to mouse Ly-41 antigen) (PC1, NPPS)) [Homo sapiens] | 11 | 6.8 | 96.83 |
| - | | - | gi\|129678\|sp\|P22413\|PC1_HUMAN PLASMA-CELL MEMBRANE GLYCOPROTEIN PC-1 [INCLUDES: ALKALINE PHOSPHODIESTERASE I ; NUCLEOTIDE PYROPHOSPHATASE (NPPASE)] | 7 | 6.8 | 99.91 |

NOTE:

1. To search again using unmatched masses, click the symbol ®.

2. Highly similar protein sequences were given the same rank (IE user: click "+" to expand/contract).

**Input Summary**

| | |
|---|---|
| **Date & Time** | Wed Feb 07 10:00:44 2001 UTC (Search Time: 5.91 sec.) |
| **Sample ID** | EDB Fibronektin, #0824, Bande 5 |
| **Database** | NCBInr [..\databases\nr] |
| **Taxonomy Category** | Mammalia (mammals) |
| **Protein Mass Range** | 80 - 100 kDa |
| **Protein pI Range** | 0.0 -14.0 |
| **Search for** | Single protein only |
| **Digest Chemistry** | Trypsin |

| | |
|---|---|
| **Max Missed Cut** | 2 |
| **Modifications** | +C3H5ON@C(Partial); +O@M(Partial); |
| **Charge State** | MH+ |
| **Peptide Masses (Da,Average)** | |
| **Tolerance(AVG)** | 1.00 ppm |
| **Peptide Masses (Da,Monoisotopic)** | 881.288 927.495 983.498 1007.525 1222.666 1376.820 1422.642 1439.854 1475.797 1479.791 1553.852 1567.742 1638.888 1781.886 1915.892 1961.078 2019.135 2044.949 2225.083 2283.131 2470.203 3143.411 3299.415 3323.912 3337.675 |
| **Tolerance(MON)** | 50.00 ppm |
| **Number of Peptides** | 25 |

ProteoMetrics' ProFound is based on ProFound at The Rockefeller University [search + transmission time: >=5.94 sec]

function expandIt(whichE1) {whichE1.style.display = (whichE1.style.display == "none")? "":"none";}

**ProFound** - Search Result Summary

Version 4.10.6

© 1997-2000 ProteoMetrics

A:hover { COLOR: red } function toggleIt(E1) {whichIm = event.srcElement;if (E1.style.display == "none"){E1.style.display = "";whichIm.src = "/prowl/minus.gif";}else{whichIm.src = "/prowl/plus.gif";E1.style.display = "none";}} A:hover { COLOR: red }

**Protein Candidates** for search 20010207110746-00D6-149234049162 [121056 sequences searched]

| Rank | Probability | Est'd Z | Protein Information and Sequence Analyse Tools (T) | % | pI | kDa |
|---|---|---|---|---|---|---|
| + 1 | 1.0e+000 | 1.61 | gi\|124963\|sp\|P05556\|ITB1_HUMAN FIBRONECTIN RECEPTOR BETA SUBUNIT PRECURSOR (INTEGRIN BETA-1) (CD29) (INTEGRIN VLA-4 BETA SUBUNIT) | 18 | 5.3 | 88.45 |
| - | | - | gi\|10336839\|gb\|AAG16767.1\|AF192528_1 (AF192528) integrin beta-1 subunit [Sus scrofa] | 12 | 5.3 | 88.25 |
| - | | - | gi\|762977\|emb\|CAA33272.1\| (X15202) Fn receptor beta prechain [Mus musculus] | 12 | 5.8 | 88.18 |
| - | | - | gi\|72070\|pir\|\|IJMSFB fibronectin receptor beta chain precursor - mouse | 12 | 5.8 | 88.31 |

| | | | | | |
|---|---|---|---|---|---|
| - | - | gi\|124964\|sp\|P09055\|ITB1_MOUSE FIBRONECTIN RECEPTOR BETA SUBUNIT PRECURSOR (INTEGRIN BETA-1) | 12 | 5.7 | 88.21 |
| - | - | gi\|8393636\|ref\|NP_058718.1\| integrin, beta 1 | 12 | 5.8 | 88.48 |
| - | - | gi\|1708573\|sp\|P53712\|ITB1_BOVIN FIBRONECTIN RECEPTOR BETA SUBUNIT (INTEGRIN BETA-1) (CD29) (INTEGRIN VLA-4 BETA SUBUNIT) | 10 | 5.3 | 85.31 |
| - | - | gi\|1708574\|sp\|P53713\|ITB1_FELCA FIBRONECTIN RECEPTOR BETA SUBUNIT PRECURSOR (INTEGRIN BETA-1) (CD29) (INTEGRIN VLA-4 BETA SUBUNIT) | 11 | 5.2 | 88.08 |
| 2 3.1e-006 | - | gi\|479805\|pir\|\|S35458 SNF2 protein homolog - human (fragment) | 12 | 7.0 | 88.59 |
| + 3 7.7e-007 | - | gi\|5725250\|emb\|CAB52406.1\| (AJ245661) G7 protein [Homo sapiens] | 8 | 5.9 | 94.65 |
| - | - | gi\|3108220\|gb\|AAC62533.1\| (AF048986) MutS homolog 5 [Homo sapiens] | 8 | 6.0 | 92.87 |
| - | - | gi\|4505253\|ref\|NP_002432.1\| mutS (E. coli) homolog 5 | 8 | 6.0 | 92.86 |
| 4 6.7e-007 | - | gi\|7512247\|pir\|\|I65253 disintegrin-like testicular metalloproteinase (EC 3.4.24.-) IVb - crab-eating macaque (fragment) | 14 | 6.6 | 80.82 |
| 5 1.8e-007 | - | gi\|10438454\|dbj\|BAB15248.1\| (AK025824) unnamed protein product [Homo sapiens] | 19 | 6.4 | 80.60 |
| 6 5.4e-008 | - | gi\|1586344\|prf\|\|2203411A reeler gene [Mus musculus] | 10 | 5.7 | 99.37 |
| 7 3.0e-008 | - | gi\|4503165\|ref\|NP_003581.1\| cullin 3 | 16 | 9.0 | 88.91 |
| + 8 1.4e-008 | - | gi\|6681275\|ref\|NP_031934.1\| eukaryotic elongation factor-2 kinase | 14 | 5.2 | 81.72 |
| - | - | gi\|6978795\|ref\|NP_037079.1\| eukaryotic elongation factor 2 kinase | 9 | 5.1 | 81.47 |
| 9 1.2e-008 | - | gi\|7662434\|ref\|NP_055733.1\| KIAA0990 protein | 15 | 9.5 | 91.71 |
| 5.2e-009 | - | gi\|7662436\|ref\|NP_055749.1\| KIAA0996 protein | 13 | 5.8 | 96.6 |

1 8

0

NOTE:

1. To search again using unmatched masses, click the symbol ®.

2. Highly similar protein sequences were given the same rank (IE user: click "+" to expand/contract).

**Input Summary**

| | |
|---|---|
| **Date & Time** | Wed Feb 07 10:07:52 2001 UTC (Search Time: 5.88 sec.) |
| **Sample ID** | EDB Fibronektin, #0824, Bande 7 |
| **Database** | NCBInr [..\databases\nr] |
| **Taxonomy Category** | Mammalia (mammals) |
| **Protein Mass Range** | 80 - 100 kDa |
| **Protein pI Range** | 0.0 -14.0 |
| **Search for** | Single protein only |
| **Digest Chemistry** | Trypsin |
| **Max Missed Cut** | 2 |
| **Modifications** | +C3H5ON@C(Partial); +O@M(Partial); |
| **Charge State** | MH+ |
| **Peptide Masses (Da,Average)** | |
| **Tolerance(AVG)** | 1.00 ppm |
| **Peptide Masses (Da,Monoisotopic)** | 881.213 983.479 1222.615 1266.561 1376.698 1422.672 1473.821 1479.786 1553.850 1567.725 1639.856 1781.886 1819.830 1915.945 1931.961 1961.051 2019.150 2068.101 2224.061 2283.101 2344.093 2470.201 2501.215 2705.264 2776.358 2840.545 2872.558 3052.493 3143.494 3159.559 3280.571 3298.572 |
| **Tolerance(MON)** | 50.00 ppm |
| **Number of Peptides** | 32 |

ProteoMetrics' ProFound is based on ProFound at The Rockefeller University [search + transmission time: >=5.91 sec]

**Patentansprüche**

**1.** Verfahren zum Screenen auf Verbindungen, die an einen Rezeptor der $ED_b$ Fibronektin - Domäne, der das $\alpha 2\beta 1$ - Integrin umfasst, binden, wobei das Verfahren umfasst:

Vergleich einer Antwort von Zellen in Anwesenheit von einer oder mehrerer dieser Verbindungen mit der Kontrollantwort besagter Zellen in Abwesenheit dieser Verbindungen, wobei die Zellen
- Proteine exprimieren, die das $\alpha 2\beta 1$ Integrin umfassen, und

wobei die Antwort bzw. die Kontrollantwort durch diesen Rezeptor der $ED_b$-Fibronektin - Domäne vermittelt wird, und wobei die Antwort bzw. die Kontrollantwort die Proliferation oder das Anhaften der Zellen an Oberflächen umfasst, die mit der $ED_b$-Fibronektin - Domäne oder Teilen davon beschichtet sind, und
wobei die Zellen ausgewählt sind aus Endothelzellen, stromalen Zellen eines Tumors und Tumorzellen.

**2.** Verfahren zum Screenen auf Verbindungen, die an die $ED_b$-Fibronektin - Domäne binden, wobei das Verfahren umfasst:

a) in Kontakt - Bringen von Zellen mit einer festgelegten Konzentration eines Proteins, das die $ED_b$-Fibronektin - Domäne oder ein Protein mit einer der in SEQ ID NO: 1 bis 4 dargestellten Sequenzen umfasst, in der Anwesenheit verschiedener Konzentrationen von einer oder mehrerer Verbindungen; und
b) Nachweis von Unterschieden in der Antwort der Zellen auf das Protein, das die $ED_b$-Fibronektin - Domäne oder ein Protein mit einer der in SEQ ID NO: 1 bis 4 dargestellten Sequenzen umfasst, in der Anwesenheit der Verbindungen im Vergleich mit der Kontrollantwort der Zellen auf das Protein, das die $ED_b$-Fibronektin - Domäne oder ein Protein mit einer der in SEQ ID NO: 1 bis 4 dargestellten Sequenzen umfasst, in der Abwesenheit dieser Verbindungen, wobei
die Zellen

- Proteine exprimieren, die das α2β1 Integrin umfassen

und wobei die Antwort bzw. die Kontrollantwort durch diesen Rezeptor der $ED_b$-Fibronektin - Domäne vermittelt wird, der das α2β1 - Integrin umfasst,
wobei die Antwort bzw. die Kontrollantwort die Proliferation oder das Anhaften der Zellen an Oberflächen umfasst, die mit der $ED_b$-Fibronektin - Domäne oder Teilen davon beschichtet sind, und
wobei die Zellen ausgewählt sind aus Endothelzellen, stromalen Zellen eines Tumors und Tumorzellen.

**3.** Verfahren zum Screenen auf Verbindungen, die an einen Rezeptor der $ED_b$-Fibronektin - Domäne, der das α2β1 - Integrin umfasst, binden, wobei das Verfahren umfasst:

Vergleich einer Antwort von Zellen in Anwesenheit von einer oder mehrerer dieser Verbindungen mit der Kontrollantwort besagter Zellen in Abwesenheit dieser Verbindungen, wobei die Zellen
Proteine exprimieren, die das α2β1 Integrin umfassen, und

wobei die Antwort bzw. die Kontrollantwort durch diesen Rezeptor der $ED_b$-Fibronektin-Domäne vermittelt wird, und wobei die Antwort bzw. die Kontrollantwort die Proliferation, Migration und Differenzierung von Endothelzellen in einer Collagenmatrix umfasst, die mit der $ED_b$-Fibronektin - Domäne oder Teilen davon versetzt wird.

**4.** Verfahren zum Screenen auf Verbindungen, die an die $ED_b$-Fibronektin - Domäne binden, wobei das Verfahren umfasst:

a) in Kontakt - Bringen von Zellen mit einer festgelegten Konzentration eines Protein, das die $ED_b$-Fibronektin - Domäne oder ein Protein mit einer der in SEQ ID NO: 1 bis 4 dargestellten Sequenzen umfasst, in der Anwesenheit verschiedener Konzentrationen von einer oder mehrerer Verbindungen; und
b) Nachweis von Unterschieden in der Antwort der Zellen auf das Protein, das die $ED_b$-Fibronektin - Domäne oder ein Protein mit einer der in SEQ ID NO: 1 bis 4 dargestellten Sequenzen umfasst, in der Anwesenheit der Verbindungen im Vergleich mit der Kontrollantwort der Zellen auf das Protein, das die $ED_b$-Fibronektin - Domäne oder ein Protein mit einer der in SEQ ID NO: 1 bis 4 dargestellten Sequenzen umfasst, in der Abwesenheit dieser Verbindungen, wobei
die Zellen

- Proteine exprimieren, die das α2β1 Integrin umfassen

und wobei die Antwort bzw. die Kontrollantwort durch diesen Rezeptor der $ED_b$-Fibronektin - Domäne vermittelt wird, der das α2β1 - Integrin umfasst, und
wobei die Antwort bzw. die Kontrollantwort die Proliferation, Migration und Differenzierung von Endothelzellen in einer Collagenmatrix umfasst, die mit der $ED_b$-Fibronektin - Domäne oder Teilen davon versetzt wird.

**5.** Verfahren nach einem der Ansprüche 1 oder 3, **dadurch gekennzeichnet, dass** die $ED_b$-Fibronektin - Domäne die Sequenz SEQ ID NO 1 - 4 oder Teile der Sequenzen SEQ ID NO 1 - 4 umfasst.

**6.** Verfahren nach einem der Ansprüche 1 bis 5, wobei die Verbindungen ausgewählt sind aus der Gruppe, die Antikörper, artifizielle Antikörper, Antikörperfragmente, Peptide, niedermolekulare Verbindungen, Aptamere und Spiegelmere umfasst.

**7.** Verfahren nach Anspruch 6, **dadurch gekennzeichnet, dass** die Antikörper rekombinante Antikörper sind.

**8.** Verfahren nach Anspruch 7, **dadurch gekennzeichnet, dass** die Antikörper ausgewählt sind aus der Gruppe, die scFv und Fragmente davon umfasst.

## Claims

1. Process of screening for compounds that bind to a receptor of the $ED_b$ fibronectin domain, which comprises the $\alpha 2\beta 1$ integrin, said process comprising:

   comparing a response by cells in the presence of one or more of said compounds with the control response by said cells in the absence of said compounds, said cells

   - expressing proteins that comprise said $\alpha 2\beta 1$ integrin, and

   wherein the response or control response is mediated by said receptor of the $ED_b$ fibronectin domain, and wherein the response or control response comprises proliferation or adhesion of the cells to surfaces coated with the $ED_b$ fibronectin domain or parts thereof, and wherein the cells are selected from the group consisting of endothelial cells, stromal cells of a tumor and tumor cells.

2. Process of screening for compounds that bind to the $ED_b$ fibronectin domain, said process comprising:

   a) bringing cells into contact with a fixed concentration of a protein that comprises the $ED_b$ fibronectin domain or a protein with one of the sequences that are represented in SEQ ID NOs: 1 to 4, in the presence of different concentrations of one or more compounds; and
   b) determination of differences in the response of cells to the protein that comprises the $ED_b$ fibronectin domain or a protein with one of the sequences that are represented in SEQ ID NOs: 1 to 4, in the presence of the compounds in comparison to the control response of cells to the protein that comprises the $ED_b$ fibronectin domain or a protein with one of the sequences that are represented in SEQ ID NOs: 1 to 4, in the absence of these compounds,
   the cells

   - expressing proteins that comprise the $\alpha 2\beta 1$ integrin, and

   wherein the response or control response is mediated by said receptor of the $ED_b$ fibronectin domain, which comprises said $\alpha 2\beta 1$ integrin,
   wherein the response or control response comprises proliferation or adhesion of the cells to surfaces coated with the $ED_b$ fibronectin domain or parts thereof, and
   wherein the cells are selected from the group consisting of endothelial cells, stromal cells of a tumor and tumor cells.

3. Process of screening for compounds that bind to a receptor of the $ED_b$ fibronectin domain, which comprises the $\alpha 2\beta 1$ integrin, said process comprising:

   comparing a response by cells in the presence of one or more of said compounds with the control response by said cells in the absence of said compounds, said cells
   expressing proteins that comprise said $\alpha 2\beta 1$ integrin, and

   wherein the response or control response is mediated by said receptor of the $ED_b$ fibronectin domain, and wherein the response or control response comprises proliferation, migration and differentiation of endothelial cells in a collagen matrix which is mixed with the $ED_b$ fibronectin domain or parts thereof.

4. Process of screening for compounds that bind to the $ED_b$ fibronectin domain, said process comprising:

   a) bringing cells into contact with a fixed concentration of a protein that comprises the $ED_b$ fibronectin domain or a protein with one of the sequences that are represented in SEQ ID NOs: 1 to 4, in the presence of different concentrations of one or more compounds; and
   b) determination of differences in the response of cells to the protein that comprises the $ED_b$ fibronectin domain or a protein with one of the sequences that are represented in SEQ ID NOs: 1 to 4, in the presence of the compounds in comparison to the control response of cells to the protein that comprises the $ED_b$ fibronectin domain or a protein with one of the sequences that are represented in SEQ ID NOs: 1 to 4, in the absence of these compounds,
   the cells

- expressing proteins that comprise the α2β1 integrin,

and wherein the response or control response is mediated by said receptor of the $ED_b$-fibronectin domain, which comprises said α2β1 integrin, and
wherein the response or control response comprises proliferation, migration and differentiation of endothelial cells in a collagen matrix which is mixed with the $ED_b$-fibronectin domain or parts thereof.

**5.** Process according to either of Claims 1 and 3, **characterized in that** the $ED_b$ fibronectin domain comprises the sequence SEQ ID NO 1-4 or parts thereof.

**6.** Process according to any of Claims 1 to 5, wherein the compounds are selected from the group comprising antibodies, artificial antibodies, antibody fragments, peptides, low molecular-weight compounds, aptamers and spiegelmers.

**7.** Process according to Claim 6, **characterized in that** the antibodies are recombinant antibodies.

**8.** Process according to Claim 7, **characterized in that** the antibodies are selected from the group comprising scFv and fragments thereof.

## Revendications

**1.** Procédé pour le criblage de composés qui se lient à un récepteur du domaine de la $ED_b$-fibronectine qui comprend l'intégrine α2β1, le procédé comprenant :

la comparaison d'une réponse de cellules en présence d'un ou de plusieurs de ces composés avec une réponse témoin desdites cellules en l'absence de ces composés,
les cellules exprimant des protéines qui comprennent l'intégrine α2β1, et
la réponse ou la réponse témoin étant provoquée par le récepteur du domaine de la $ED_b$-fibronectine, et
la réponse ou la réponse témoin comprenant la prolifération ou l'adhérence des cellules aux surfaces qui sont revêtues du domaine de la $ED_b$-fibronectine ou de parties de celui-ci, et
les cellules étant choisies parmi les cellules endothéliales, les cellules stromales d'une tumeur et les cellules tumorales.

**2.** Procédé pour le criblage de composés qui se lient au domaine de la $ED_b$-fibronectine, le procédé comprenant :

a) la mise en contact de cellules avec une concentration définie en une protéine qui comprend le domaine de la $ED_b$-fibronectine ou une protéine qui comprend une des séquences représentées dans les SEQ ID NO : 1 à 4, en présence de différentes concentrations en un ou plusieurs composés ; et
b) la détection de différences dans la réponse des cellules à la protéine qui comprend le domaine de la $ED_b$-fibronectine ou à une protéine qui comprend une des séquences représentées dans les SEQ ID NO : 1 à 4, en présence des composés par rapport à la réponse témoin des cellules à la protéine qui comprend le domaine de la $ED_b$-fibronectine ou à une protéine qui comprend une des séquences représentées dans les SEQ ID NO : 1 à 4, en l'absence de ces composés,
les cellules exprimant des protéines qui comprennent l'intégrine α2β1, et
la réponse ou la réponse témoin étant provoquée par le récepteur du domaine de la $ED_b$-fibronectine qui comprend l'intégrine α2β1,
la réponse ou la réponse témoin comprenant la prolifération ou l'adhérence des cellules aux surfaces qui sont revêtues du domaine de la $ED_b$-fibronectine ou de parties de celui-ci, et
les cellules étant choisies parmi les cellules endothéliales, les cellules stromales d'une tumeur et les cellules tumorales.

**3.** Procédé pour le criblage de composés qui se lient à un récepteur du domaine de la $ED_b$-fibronectine qui comprend l'intégrine α2β1, le procédé comprenant :

la comparaison d'une réponse de cellules en présence d'un ou de plusieurs de ces composés avec une réponse témoin desdites cellules en l'absence de ces composés,
les cellules exprimant des protéines qui comprennent l'intégrine α2β1, et
la réponse ou la réponse témoin étant provoquée par 1 e récepteur du domaine de la $ED_b$-fibronectine, et la réponse ou la réponse témoin comprenant la prolifération, la migration et la différenciation des cellules endothéliales dans une matrice de collagène, qui est mélangée avec le domaine de la $ED_b$-fibronectine ou des parties de celui-ci.

**4.** Procédé pour le criblage de composés qui se lient à un récepteur du domaine de la $ED_b$-fibronectine, le procédé comprenant :

a) la mise en contact de cellules avec une concentration définie en une protéine qui comprend le domaine de la $ED_b$-fibronectine ou une protéine qui comprend une des séquences représentées dans la SEQ ID NO : 1 à 4, en présence de différentes concentrations en un ou plusieurs composés ; et
b) la détection de différences dans la réponse des cellules à la protéine qui comprend le domaine de la $ED_b$-fibronectine ou à une protéine qui comprend une des séquences représentées dans les SEQ ID NO : 1 à 4, en présence des composés par rapport à la réponse témoin des cellules à la protéine qui comprend le domaine de la $ED_b$-fibronectine ou à une protéine qui comprend une des séquences représentées dans les SEQ ID NO : 1 à 4, en l'absence de ces composés,

les cellules exprimant des protéines qui comprennent l'intégrine $\alpha2\beta1$, et
la réponse ou la réponse témoin étant provoquée par le récepteur du domaine de la $ED_b$-fibronectine qui comprend l'intégrine $\alpha2\beta1$, et
la réponse ou la réponse témoin comprenant la prolifération, la migration et la différenciation des cellules endothéliales dans une matrice de collagène, qui est mélangée avec le domaine de la $ED_b$-fibronectine ou des parties de celui-ci.

**5.** Procédé selon l'une quelconque des revendications 1 ou 3, **caractérisé en ce que** le domaine de la $ED_b$-fibronectine comprend les séquences SEQ ID NO 1 - 4 ou des parties des séquences SEQ ID NO 1- 4.

**6.** Procédé selon l'une quelconque des revendications 1 à 5, les composés étant choisis dans le groupe qui comprend les anticorps, les anticorps artificiels, les fragments d'anticorps, les peptides, les composés de bas poids moléculaire, les aptamères et les Spiegelmères.

**7.** Procédé selon la revendication 6, **caractérisé en ce que** les anticorps sont des anticorps recombinants.

**8.** Procédé selon la revendication 7, **caractérisé en ce que** les anticorps sont choisis dans le groupe qui comprend les scFv et les fragments de ceux-ci.

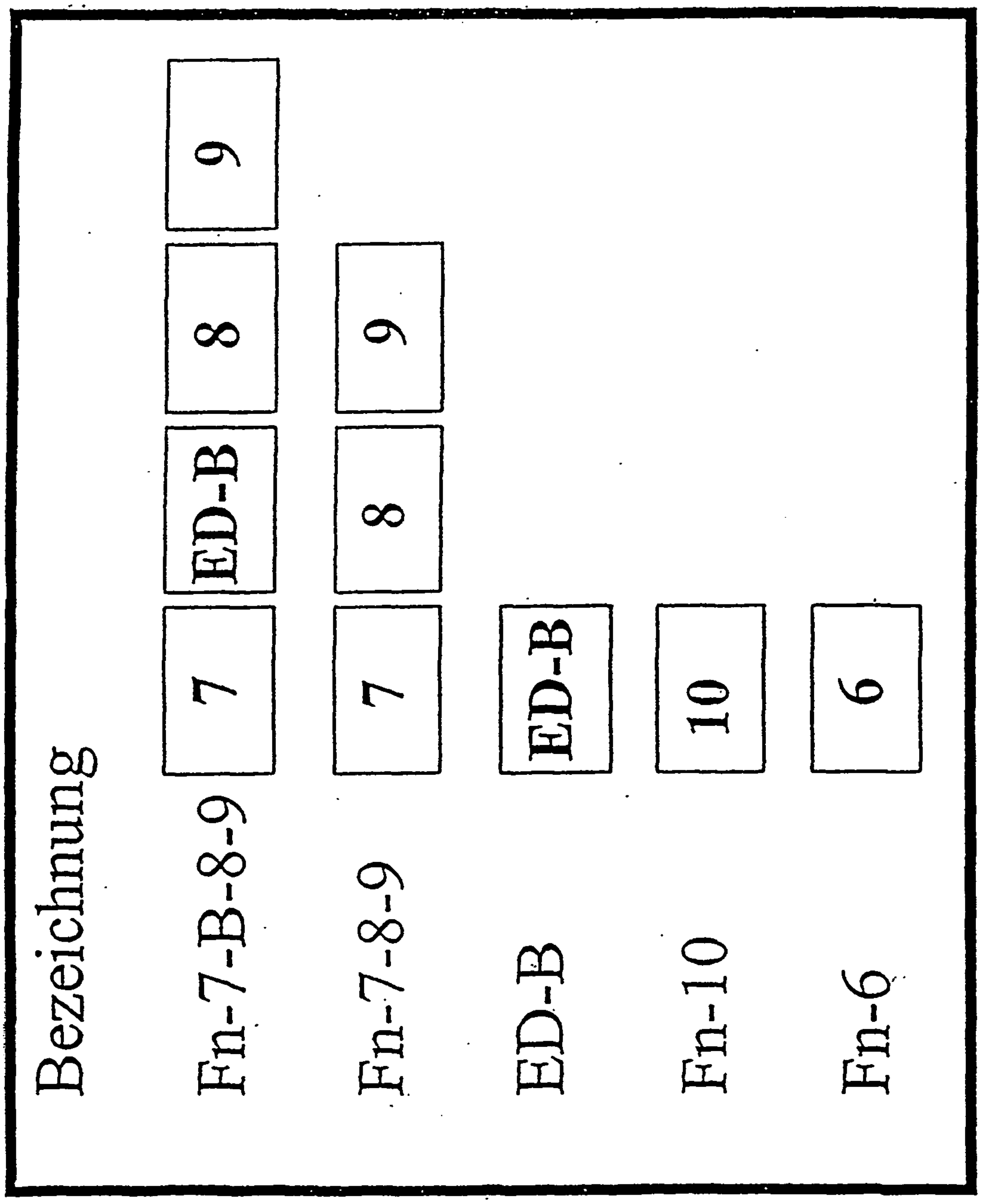


Fig. 1

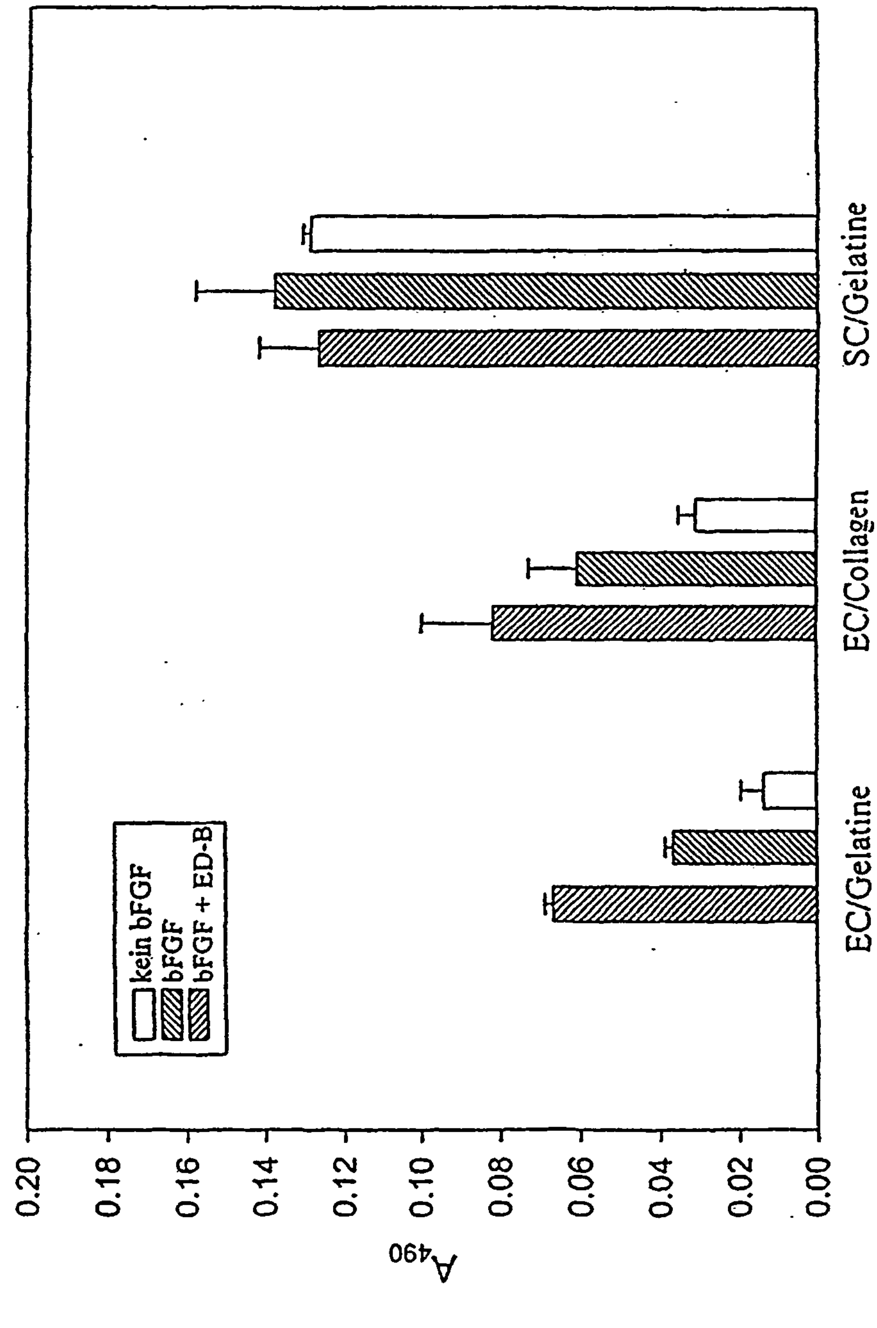
kein bFGF
bFGF
bFGF + ED-B
EC/Gelatine
EC/Collagen
SC/Gelatine
0.20
0.18
0.16
0.14
0.12
0.10
0.08
0.06
0.04
0.02
0.00
$A_{490}$

Fig. 2

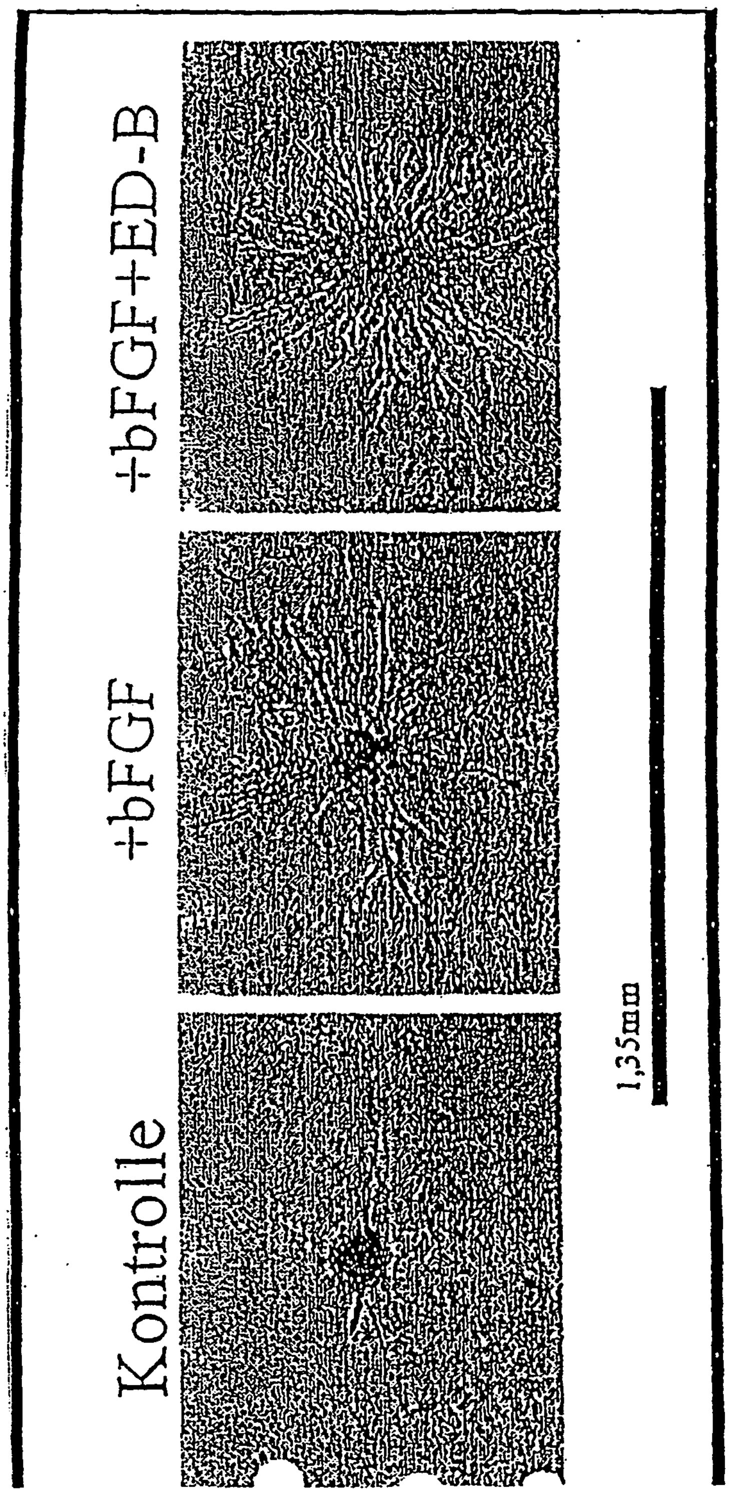


Fig. 3

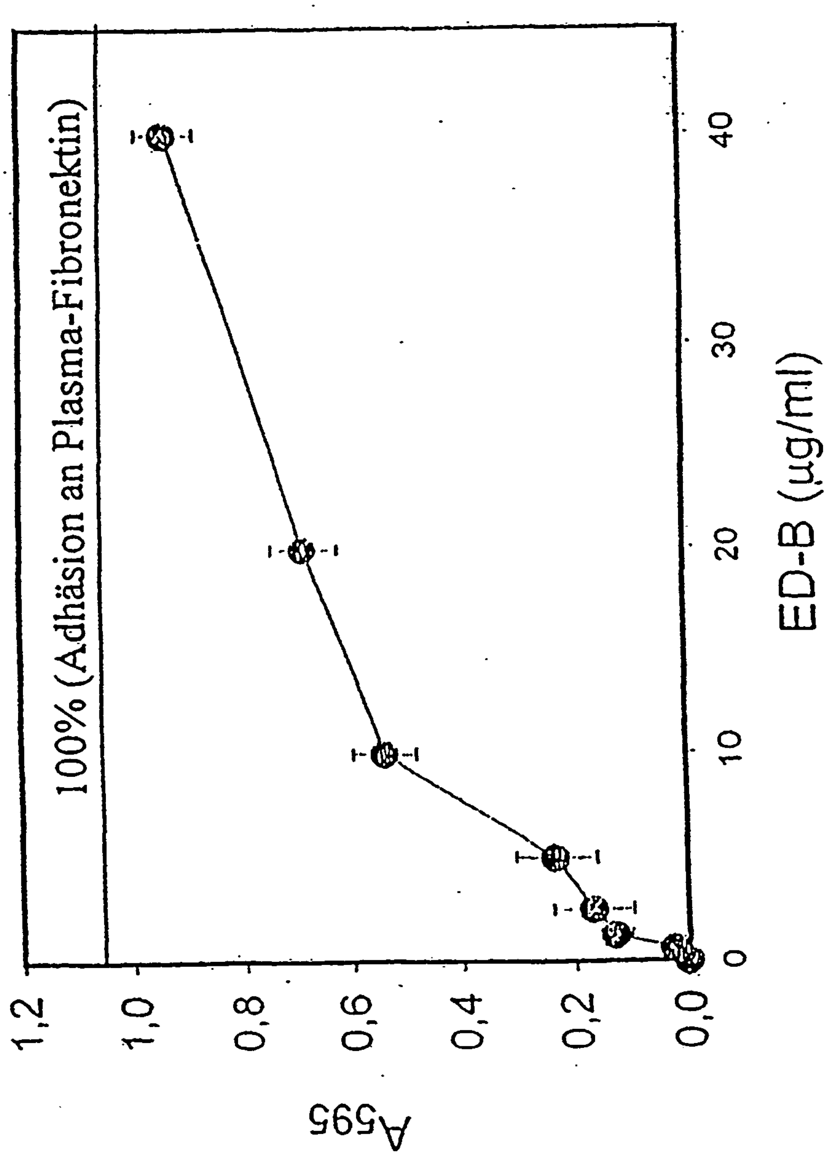
100% (Adhäsion an Plasma-Fibronektin)
$A_{595}$
1,2
1,0
0,8
0,6
0,4
0,2
0,0
0
10
20
30
40
ED-B (µg/ml)

Fig. 4

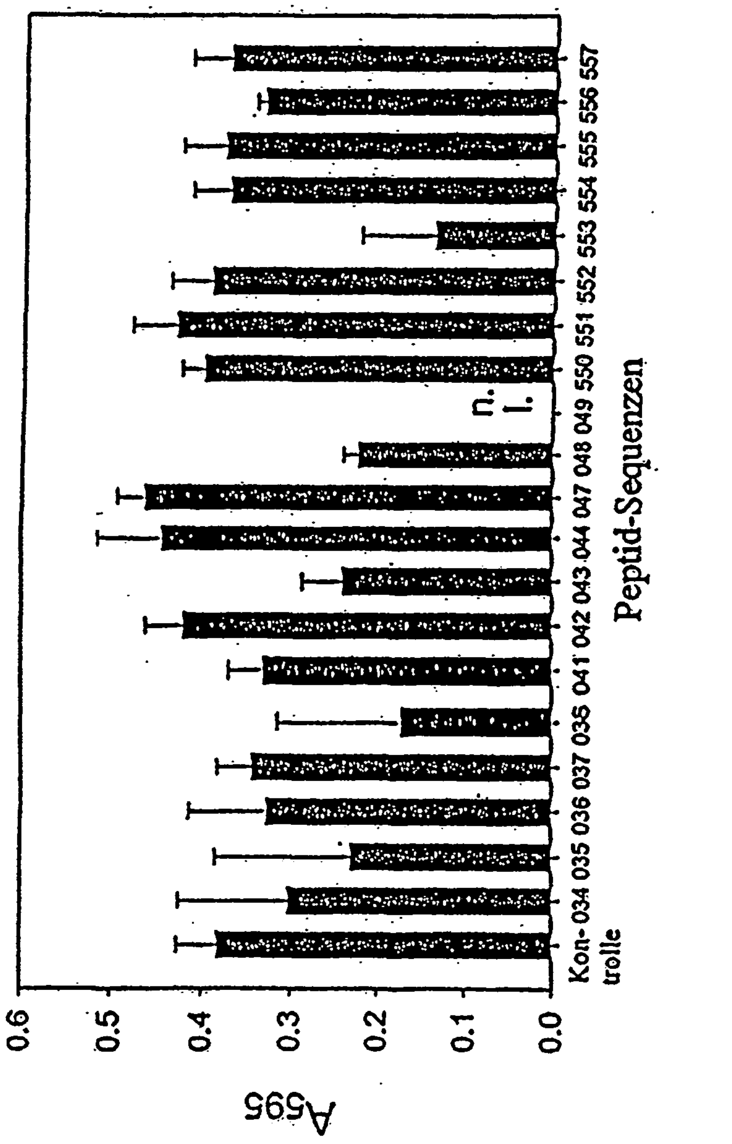


n. l. = nicht löslich

Fig. 5

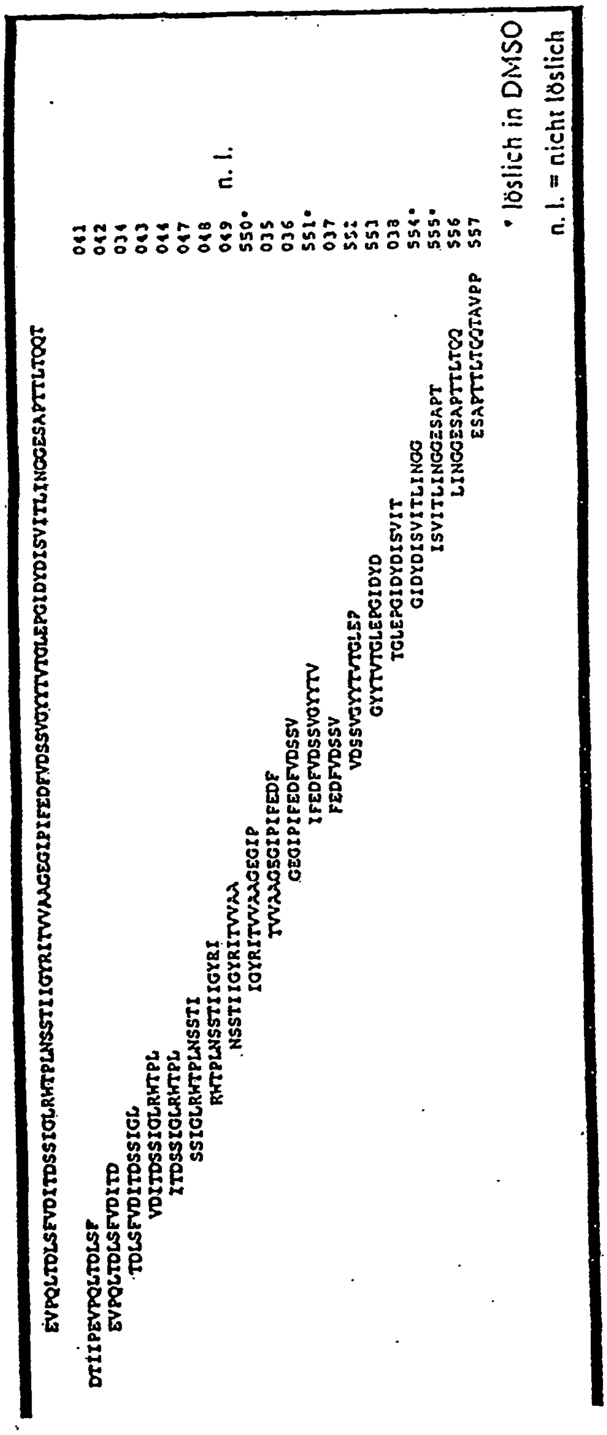
n. l.
* löslich in DMSO
n. l. = nicht löslich

Fig. 6

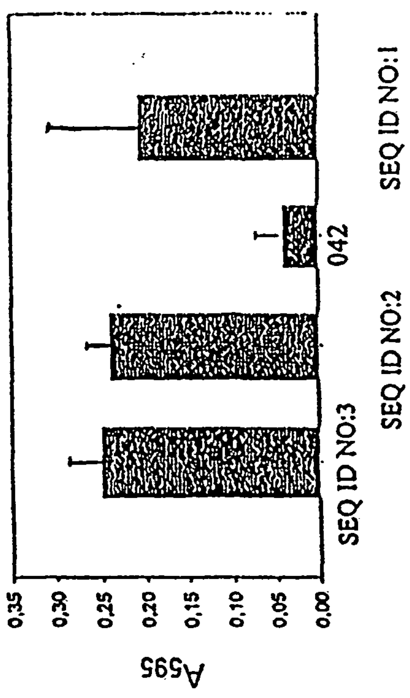
A595
0,35
0,30
0,25
0,20
0,15
0,10
0,05
0,00
SEQ ID NO:3
SEQ ID NO:2
042
SEQ ID NO:1

Fig. 7

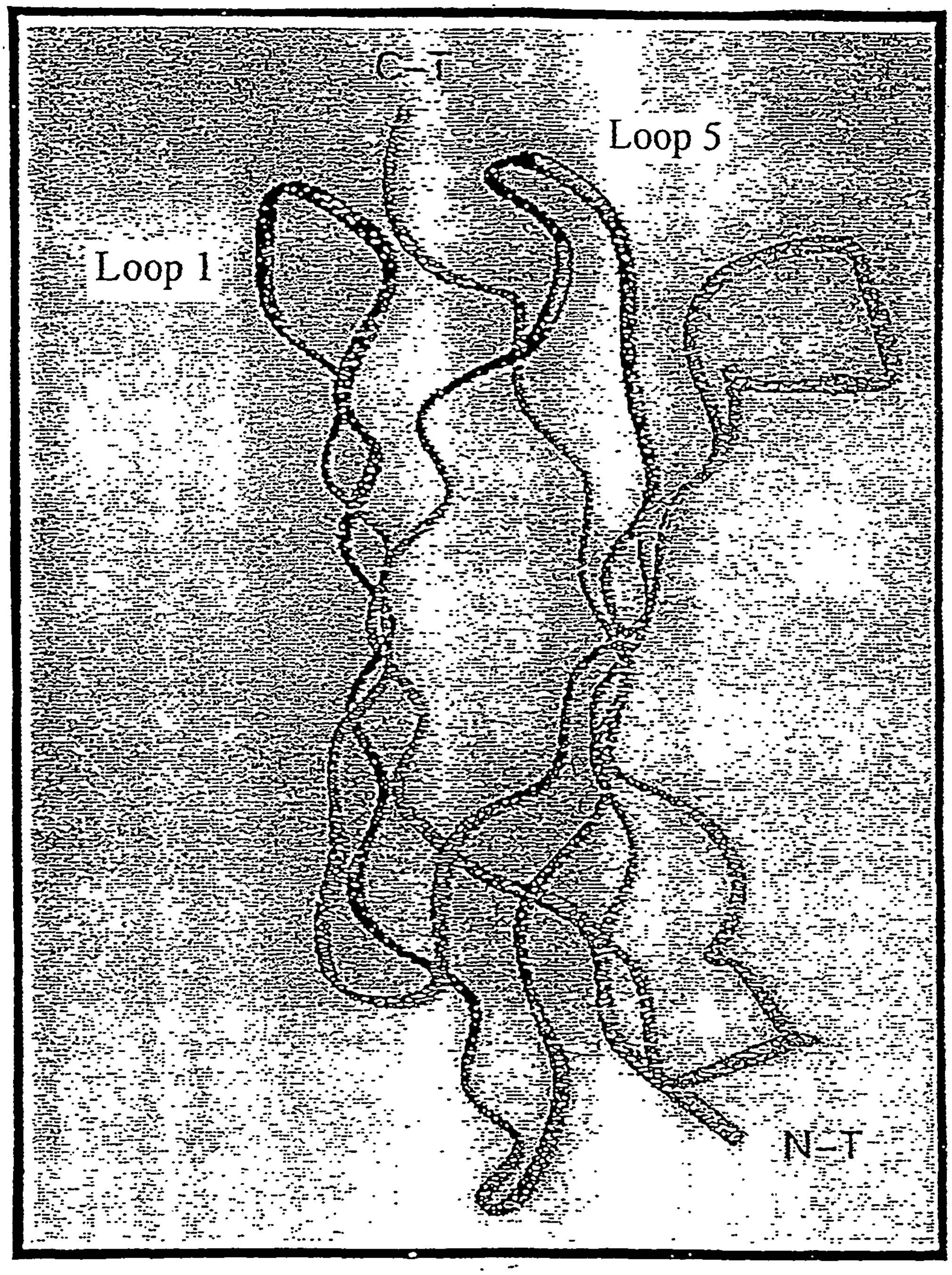


Fig. 8

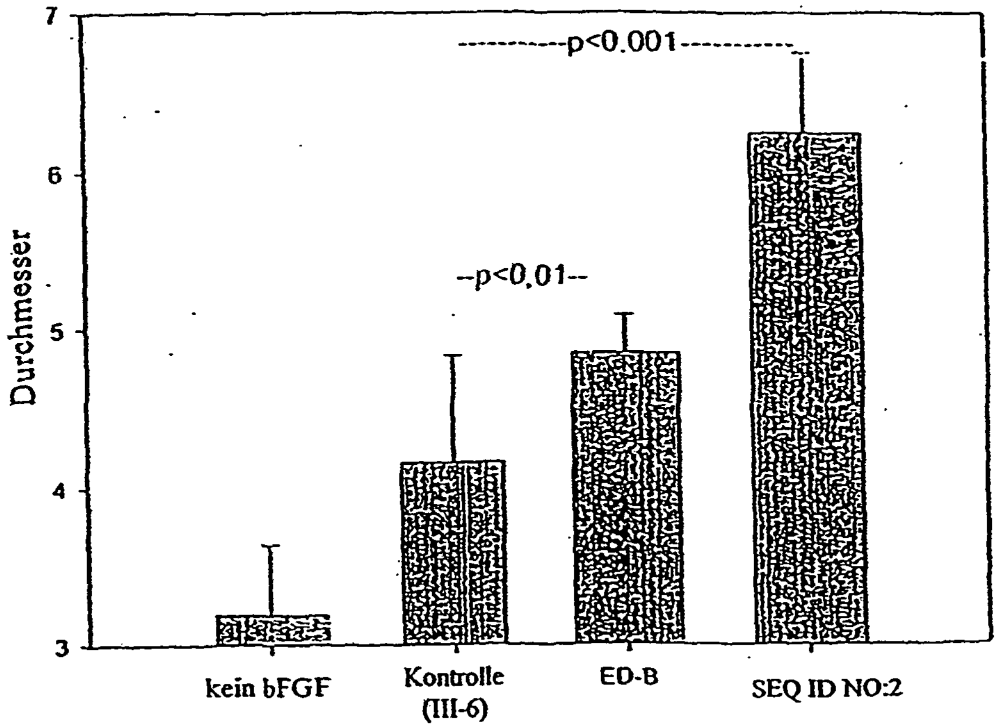
7
6
5
4
3
Durchmesser
--p<0.01--
--------p<0.001----------
kein bFGF
Kontrolle (III-6)
ED-B
SEQ ID NO:2

Fig. 9

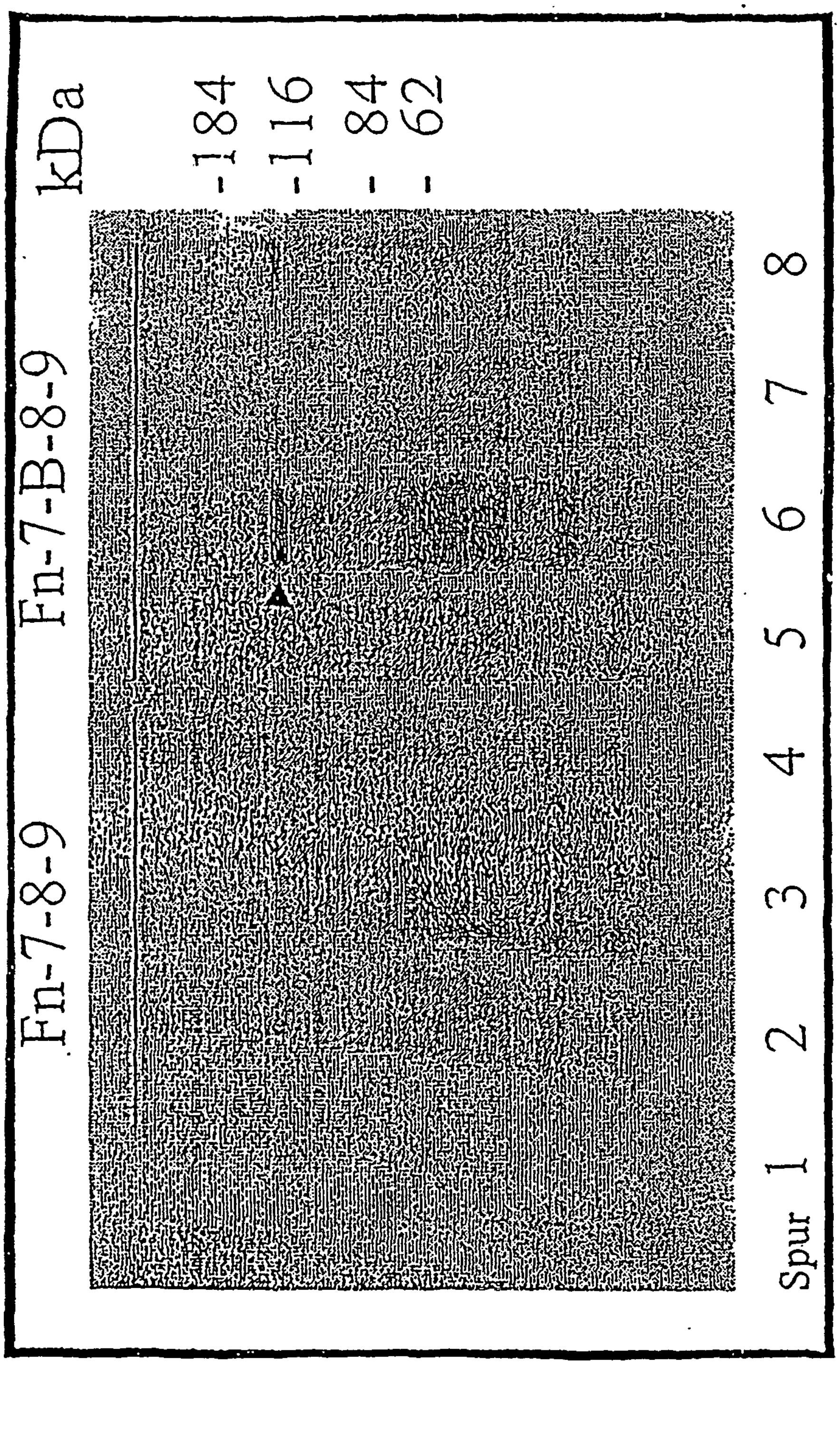


Fig. 10

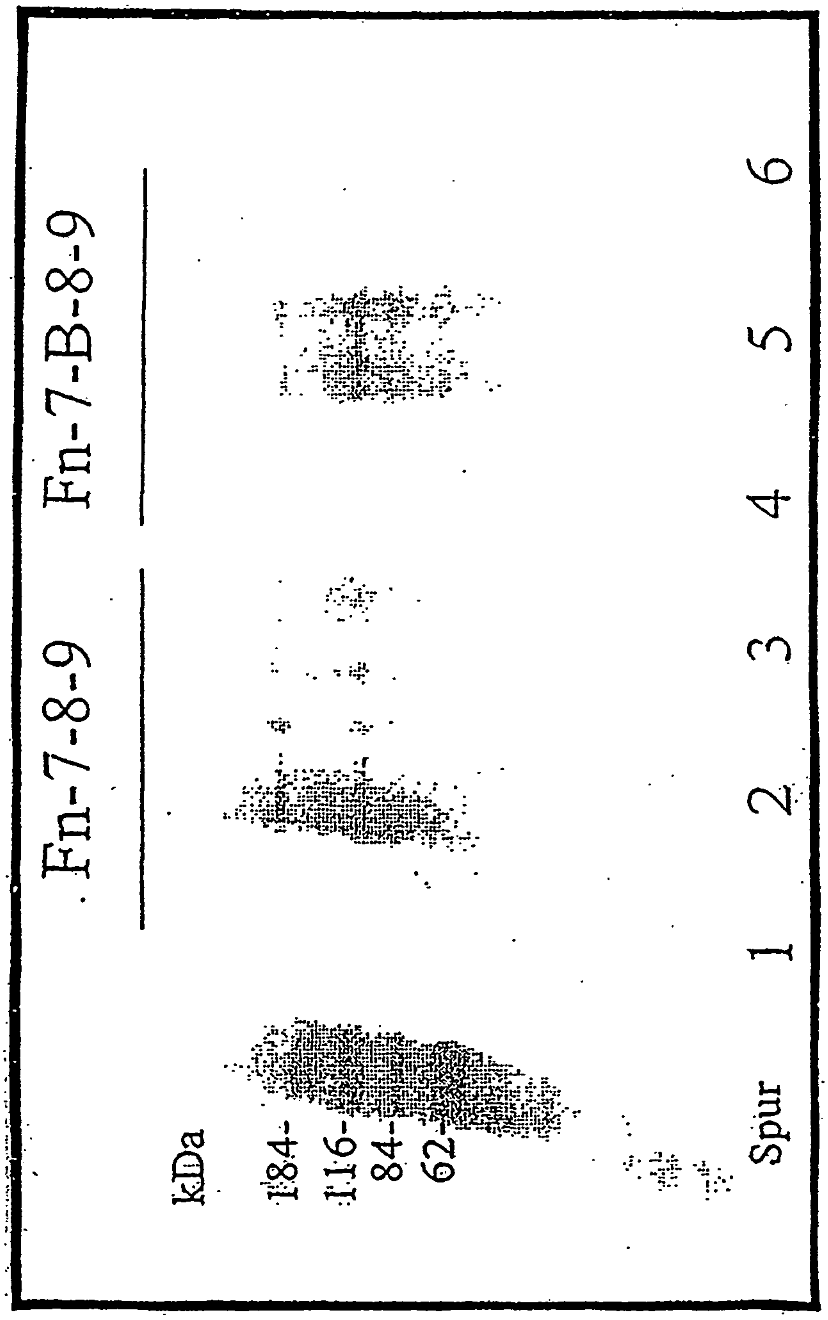


Fig. 11

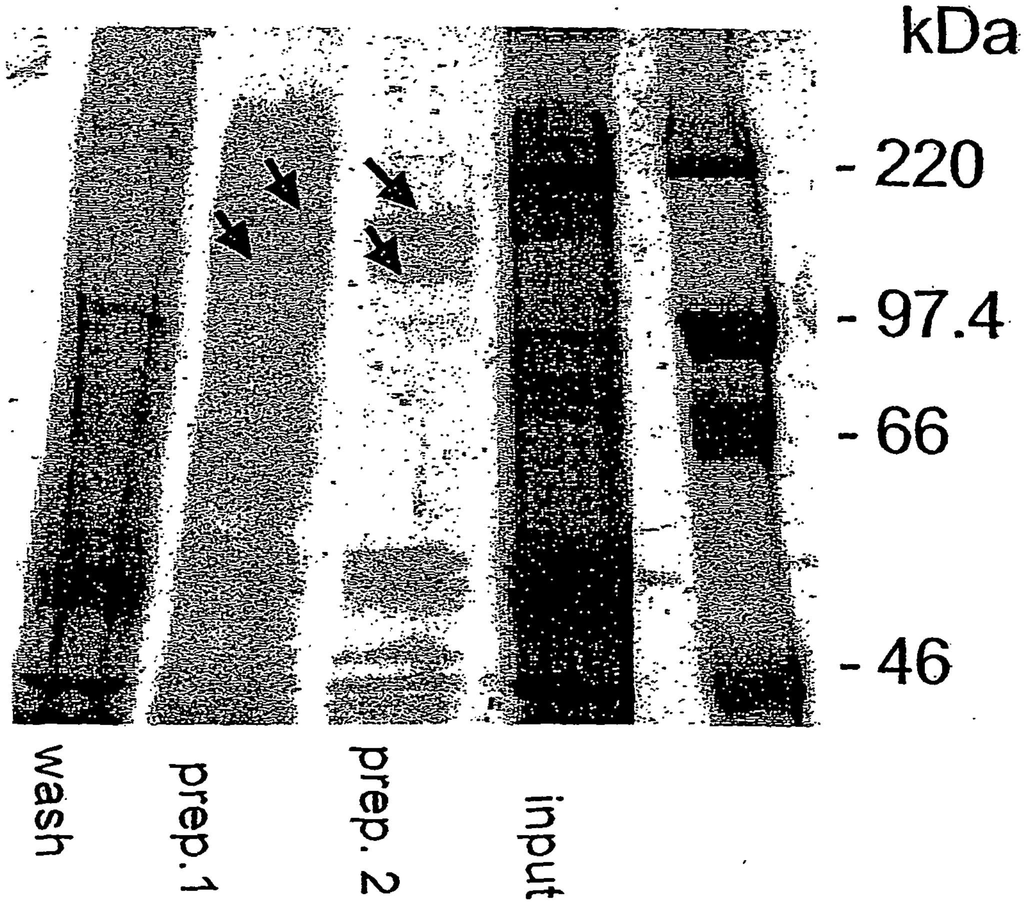


Fig. 12

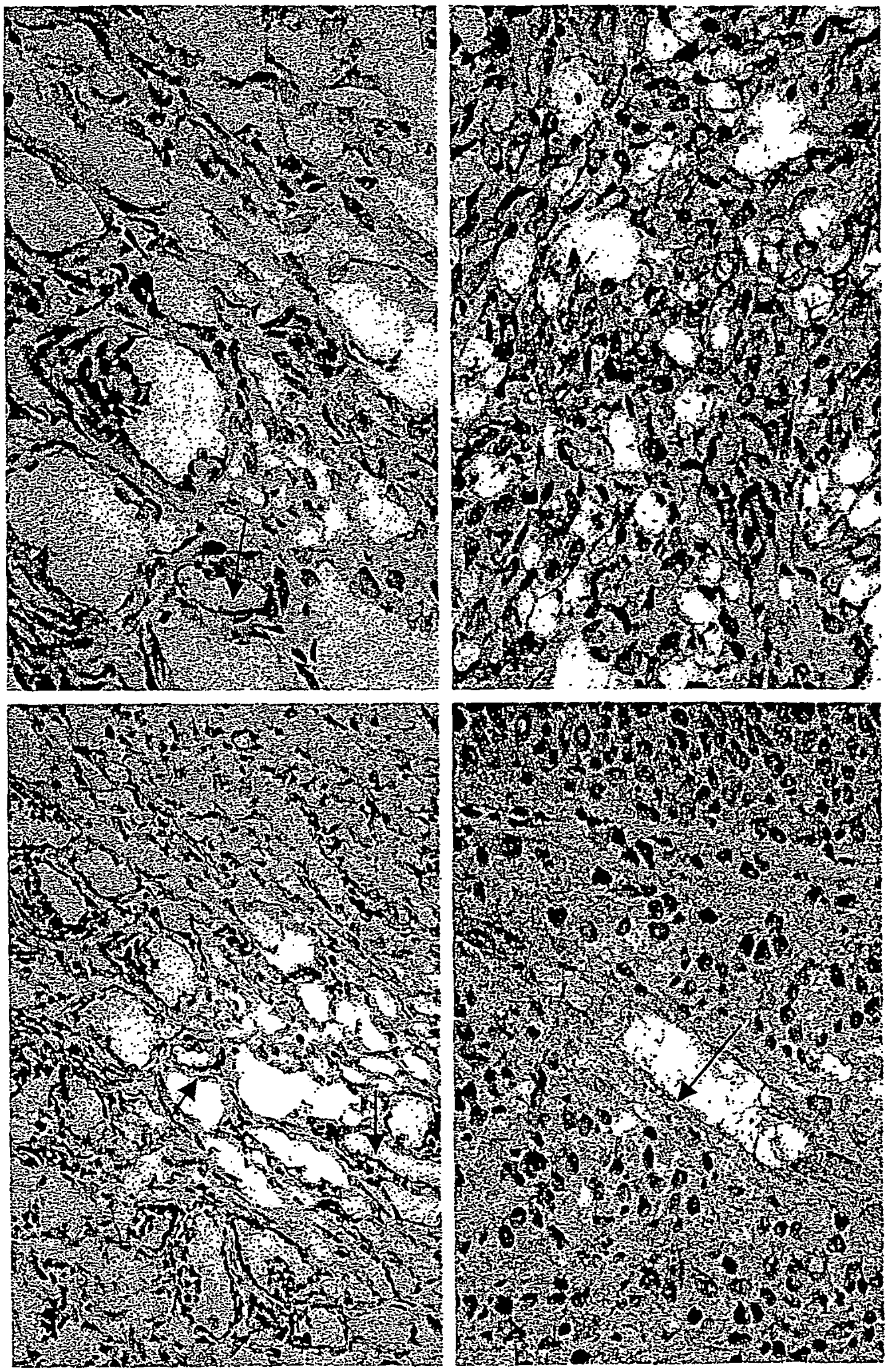

Fig. 13

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **KOMBLIHTT AR ; VIBE-PEDERSEN K ; BARALLE FE.** Isolation and characterization of cDNA clones for human and bovine fibronectins. *Proc Natl Acad Sci USA,* 1983, vol. 80, 3218-22
- **LEAHY DJ ; HENDRICKSON WA ; AUKHIL I ; ERICKSON HP.** Structure of fibronectin type III domain from tenascin phased by MAD analysis of the selenomethionyl protein. *Science,* 1992, vol. 258, 987-91
- **HYNES R.O.** *Cell,* 1987, vol. 48, 549-550
- **PLOW E. F. et al.** *J Biol Chem,* 2000, vol. 275, 21785-21788
- **JARNAGIN W. ; ROCKEY D. ; KOTELIANSKY V. ; WANG S. ; BISSELL D.** Expression of variant fibronectins in wound healing: cellular source and biological activitiy of the EIIIA segment in rat hepatic fibrogenesis. *J Cell Biol,* 1994, vol. 127, 2037-4.8
- **CASTELLANI P ; VIALE G ; DORCARATTO A ; NICOLO G ; KACZMAREK J ; QUERZE G ; ZARDI L.** *Int. J. Cancer,* 1994, vol. 59, 612-618
- **CHEN ; CULP.** *Exp. Cells Res.,* 1996, vol. 223, 9-19
- **CHEN ; CULP.** *Clin. Exp. Metast.,* 1998, vol. 16 (1), 30-42
- **XING Z ; CHEN HC ; NOWLEN JK ; TAYLOR SJ ; SHALLOWAY D ; GUAN JL.** Direct interaction of v-Src with the focal adhesion kinase mediated by the Src SH2 domain. *Mol Biol Cell,* 1994, vol. 5, 413-21
- **HUNTER T ; VAN DER GEER P.** Integrin-mediated signal transduction linked to Ras pathway by GRB2 binding to focal adhesion kinase. *Nature,* 1994, vol. 372, 786-91
- **CHEN HC ; GUAN JL.** Association of focal adhesion kinase with its potential substrate phosphatidylinositol 3-kinase. *Proc Natl Acad Sei USA,* 1994, vol. 91, 10148-52
- **SAKAI R. LWAMATSU ; A. HIRANO N et al.** A novel signaling molecule, p130, forms stable complexes in vivo with v-Crk and c-Src in a tyrosine phosphorylation-dependent manner. *EMBO J.,* 1994, vol. 13, 3748-56
- **PETCH LA ; BOCKHOLT SM ; BOUTON A ; PARSONS JT ; BURRIDGE K.** Adhesion-induced tyrosine phosphorylation of the p130 SRC substrate. *J Cell Sci,* 1995, vol. 108, 1371-9
- **POLTE TR ; HANKS SK.** Interaction between focal adhesion kinase and Crk-associated tyrosine kinase substrate p130. *Proc Natl Acad Sci USA,* 1995, vol. 92, 10678-82
- **NOBES CD ; HALL A.** Rho, rac, und cd42 GTPases regulate the assembly of multimolecular focal complexes associated with actin stress fibers, lamellipodia, and filopodia. *Cell,* 1995, vol. 81, 53-62
- **HASHIMOTO-UOSHIMA et al.** *J. Cell Sci.,* 1997, vol. 110, 2271-2280

SEQUENZPROTOKOLL

<110> Schering AG

<120> Rezeptor der $ED_b$-Fibronektin-Domäne

<130> s5495

<140>

<141>

<160> 4

<170> Patentin Ver. 2.1

<210> 1
<211> 15
<212> PRT
<213> Bindungssequenz Nr. I für den putativen EDB-Rezeptor auf dem EDB-Molekül

<400> 1

Val Asp Ile Thr Asp Ser Ser Ile Gly Leu Arg Trp Thr Pro Leu
1 5 10 15

<210> 2
<211> 15
<212> PRT
<213> Bindungssequenz Nr. II für den putativen EDB-Rezeptor auf dem EDB-Molekül

<400> 2

Gly Tyr Tyr Thr Val Thr Gly Leu Glu Pro Gly Ile Asp Tyr Asp
1 5 10 15

<210> 3
<211 > 15
<212> PRT
<213> Bindungssequenz Nr. III für den putativen EDB-Rezeptor auf dem EDB-Molekül

<400> 3

Thr Gly Leu Glu Pro Gly Ile Asp Tyr Asp Ile Ser Val Ile Thr
1 5 10 15

<210> 4
<211> 91
<212> PRT
<213> homo sapiens

<400> 4

Glu Val Pro Gln Leu Thr Asp Leu Ser Phe Val Asp Ile Thr Asp Ser
1 5 10 15

Ser Ile Gly Leu Arg Trp Thr Pro Leu Asn Ser Ser Thr Ile Ile Gly
20 25 30

Tyr Arg Ile Thr Val Val Ala Ala Gly Glu Gly Ile Pro Ile Phe Glu
35 40 45

Asp Phe Val Asp Ser Ser Val Gly Tyr Tyr Thr Val Thr Gly Leu Glu
50 55 60

Pro Gly Ile Asp Tyr Asp Ile Ser Val Ile Thr Leu Ile Asn Gly Gly
65 70 75 80

Glu Ser Ala Pro Thr Thr Leu Thr Gln Gln Thr
85 90